# EUROPEAN PATENT APPLICATION

(11) **EP 2 337 134 A1**
(43) Date of publication of application: **22.06.2011**
(21) Application number: 09819219.8
(22) Date of filing: 07.10.2009
(51) Int. Cl.: H01M 8/02, H01M 4/90, H01M 8/16

(54) **FUEL CELL, ELECTRONIC DEVICE AND BUFFER SOLUTION FOR FUEL CELL**

(30) Priority: 09.10.2008 JP 2008262592
(71) Applicant: Sony Corporation, Tokyo 108-0075 (JP)
(72) Inventor: KUMITA Hideyuki, Tokyo 108-0075 (JP); MITA Hiroki, Tokyo 108-0075 (JP); GOTO Yoshio, Tokyo 108-0075 (JP); NAKAGAWA Takaaki, Tokyo 108-0075 (JP); SAKAI Hideki, Tokyo 108-0075 (JP); MATSUMOTO Ryuhei, Tokyo 108-0075 (JP); TOKITA Yuichi, Tokyo 108-0075 (JP)
(74) Representative: Müller - Hoffmann & Partner
(86) International application number: PCT/JP2009/067489
(87) International publication number: WO 2010/041685

(57) **Abstract**

A fuel cell with which in the case where an enzyme is immobilized to one or both of a cathode and an anode, sufficient buffer ability is able to be obtained even at the time of high output operation, ability inherent in the enzyme is able to be sufficiently demonstrated, and which has superior performance is provided. In a bio-fuel cell which has a structure in which a cathode 2 and an anode 1 are opposed to each other with an electrolyte layer 3 containing a buffer substance in between, and in which an enzyme is immobilized to one or both of the cathode 2 and the anode 1, a compound containing an imidazole ring is contained in the electrolyte layer 3 as a buffer substance, and one or more acids selected from the group consisting of acetic acid, phosphoric acid, and sulfuric acid are further added. As the compound containing an imidazole ring, imidazole, 1-methylimidazole, 2-methylimidazole, 4-methylimidazole, and 2-ethylimidazole or the like is used.

## Description

### Technical Field

The present invention relates to a fuel cell in which an enzyme is immobilized to one or both of a cathode and an anode, an electronic device using the fuel cell, and a buffer solution for a fuel cell suitably used for the fuel cell.

### Background Art

The fuel cell has a structure in which a cathode (oxidant electrode) and an anode (fuel electrode) are opposed to each other with an electrolyte (proton conductor) in between. In the existing fuel cell, a fuel (hydrogen) supplied to the anode is oxidized and separated into electrons and protons (H⁺). The electrons are delivered to the anode, and H⁺ is moved to the cathode through the electrolyte. In the cathode, such H⁺ is reacted with externally supplied oxygen and the electrons sent from the anode through an external circuit, and therefore H₂O is generated.

As described above, the fuel cell is a highly effective power generating equipment that directly converts chemical energy of the fuel to electric energy. In addition, the fuel cell is able to extract chemical energy of fossil energy such as natural gas, petroleum, and coal as electric energy with high conversion efficiency without relation to usage location and usage time. Thus, in the past, research and development of the fuel cell for the purpose of large-scale power generation or the like has been actively made. For example, when the fuel cell was mounted on space shuttles, it was demonstrated that the fuel cell was able to provide not only electric power but also water for crews, and that the fuel cell was a clean power generating equipment.

Further, in recent years, fuel cells such as a polymer electrolyte fuel cell in which the working temperature range is comparatively low, for example, from room temperature to about 90 deg C both inclusive have been developed and have attracted attentions. Thus, it has been considered to apply the fuel cell not only to the large-scale power generation, but also to a small system such as a power source for driving a car and a portable power source for a personal computer, a mobile device and the like.
As described above, the fuel cell is possibly used in broad areas from the large-scale power generation to the small-scale power generation, and attracts many attentions as a highly effective power generating equipment. However, in the fuel cell, as a fuel, in general, natural gas, petroleum, coal or the like is converted to hydrogen gas with the use of a reformer. Thus, there has been a problem that limited resources are consumed. Further, there has been following problems as well. The fuel cell should be heated to high temperature, and needs an expensive precious metal catalyst such as platinum (Pt). Further, in the case where hydrogen gas or methanol is directly used as a fuel, care should be exercised in handling thereof.

Thus, it has been proposed that biologic metabolism in living matters is applied to the fuel cell by focusing attention on the fact the biologic metabolism in living matters is a highly effective energy conversion mechanism. The biologic metabolism herein includes aspiration, photonic synthesis and the like in microbial body cells. The biologic metabolism has significantly high power generation efficiency. In addition, the biologic metabolism also has an advantage that reaction proceeds under moderate conditions such as about room temperature.
For example, aspiration is a mechanism in which nutrition such as sugars, fat, and protein is taken in a microorganism or a cell, and the chemical energy thereof is converted to electric energy as follows. That is, carbon dioxide (CO₂) is generated from the taken nutrition through a glycolysis system cycle and a tricarboxylic acid (TCA) cycle having various enzyme reaction steps. In the course of generating the carbon dioxide, nicotinamide adenine dinucleotide (NAD⁺) is reduced to reduced nicotinamide adenine dinucleotide (NADH). Thereby, the chemical energy is converted to redox energy, that is, electric energy. Further, in electron transfer system, such electric energy of NADH is directly converted to proton gradient electric energy, oxygen is reduced, and water is generated. Due to the electric energy herein obtained, adenosine triphosphate (ATP) is generated from adenosine diphosphate (ADP) through ATP synthesis enzyme. Such ATP is used for reaction needed for growing the microorganism or the cell. Such energy conversion is generated in a cytosol and a mitochondria.

Further, photonic synthesis is a mechanism in which light energy is taken in, nicotinamide adenine dinucleotide phosphate (NADP⁺) is reduced to reduced nicotinamide adenine dinucleotide phosphate (NADPH) through electron transfer system, and therefore light energy is converted to electric energy, in which water is oxidized and oxygen is generated. Such electric energy is used for taking in CO₂ and carbon immobilization reaction, and is used for synthesizing carbon hydrate.
As a technology for using the foregoing biologic metabolism for the fuel cell, a microbial battery in which electric energy generated in a microorganism is taken out of the microorganism through an electron mediator, the electron is delivered to an electrode, and therefore a current is obtained has been reported (for example, refer to Patent Document 1).

However, in microorganisms and cells, many unnecessary reactions other than the intended reaction that is converting chemical energy to electric energy exist. Thus, in the foregoing method, electric energy is consumed by undesired reaction, and sufficient energy conversion efficiency is not demonstrated.
Thus, a fuel cell (bio-fuel cell) in which only desired reaction is initiated by using an enzyme has been proposed (for example, refer to Patent Documents 2 to 10). In the bio-fuel cell, a fuel is degraded by the enzyme and is thereby separated into protons and electrons. The bio-fuel cell in which alcohol such as methanol and ethanol is used as a fuel or the bio-fuel cell in which a monomeric sugar such as glucose is used as a fuel have been developed.

In the bio-fuel cell, in general, a buffer substance (buffer solution) is contained in an electrolyte for the following reason. That is, since the enzyme used as a catalyst is significantly sensitive to pH of a solution, pH value is controlled to around the value at which the enzyme easily functions by the buffer substance. In the past, as a buffer substance, sodium dihydrogen phosphate (NaH₂PO₄), 3-(N-morpholino)propanesulfonic acid (MOPS), N-2-hydroxyethyl piperazine-N'-2-ethane sulfonic acid (HEPES) or the like has been used. The concentration of the buffer substance has been 0.1 M or less generally for the following reason. That is, it has been general that the concentration of the buffer substance is diluted as much as possible down to the minimum necessary for uniformalizing pH, and appropriate inorganic ions and appropriate organic ions are added to obtain a state close to physiological condition.

### Citation list

### Patent documents

Patent document 1: Japanese Unexamined Patent Application Publication No. 2000-133297
Patent document 2: Japanese Unexamined Patent Application Publication No. 2003-282124
Patent document 3: Japanese Unexamined Patent Application Publication No. 2004-71559
Patent document 4: Japanese Unexamined Patent Application Publication No. 2005-13210
Patent document 5: Japanese Unexamined Patent Application Publication No. 2005-310613
Patent document 6: Japanese Unexamined Patent Application Publication No. 2006-24555
Patent document 7: Japanese Unexamined Patent Application Publication No. 2006-49215
Patent document 8: Japanese Unexamined Patent Application Publication No. 2006-93090
Patent document 9: Japanese Unexamined Patent Application Publication No. 2006-127957
Patent document 10: Japanese Unexamined Patent Application Publication No. 2006-156354

### Summary of the Invention

However, according to researches by the inventors of the present invention, in the foregoing existing bio-fuel cell in which NaH₂PO₄, MOPS, HEPES or the like is used as a buffer substance contained in the electrolyte, the following problem has existed. That is, in the existing bio-fuel cell, in the case where high output is realized by immobilizing an enzyme onto an electrode with a large surface area such as porous carbon or increasing the concentration of the enzyme to be immobilized onto the electrode, buffer ability is not sufficient, and pH of the electrolyte around the enzyme is shifted from the optimum pH. Accordingly, ability inherent in the enzyme has not been demonstrated sufficiently.

In view of the foregoing problems, it is an object of the present invention to provide a fuel cell with which at the time of high output operation, sufficient buffer ability is able to be obtained, ability inherent in an enzyme is able to be sufficiently demonstrated, and superior performance is demonstrated in the case where the enzyme is immobilized to one or both of a cathode and an anode.
It is another object of the present invention to provide an electronic device using the foregoing superior fuel cell.
It is still another object of the present invention to provide a buffer solution for a fuel cell suitably used for a fuel cell which has a structure in which a cathode and an anode are opposed to each other with an electrolyte containing a buffer substance in between, and in which an enzyme is immobilized to one or both of the cathode and the anode.

To resolve the foregoing problem, a first aspect in accordance with the present invention provides a fuel cell having a structure in which a cathode and an anode are opposed to each other with an electrolyte containing a buffer substance in between, wherein an enzyme is immobilized to one or both of the cathode and the anode, and a compound containing an imidazole ring is contained in the buffer substance.

Specific examples of the compound containing an imidazole ring include imidazole, tirazole, a pyridine derivative, a bipyridine derivative, and an imidazole derivative (histidine, 1-methylimidazole, 2-methylimidazole, 4-methylimidazole, 2-ethylimidazole, imidazole-2-carboxylic acid ethyl, imidazole-2-carboxyaldehyde, imidazole-4-carboxylic acid, imidazole-4,5-dicarboxylic acid, imidazole-1-yl-acetic acid, 2-acetylbenzimidazole, 1-acetylimidazole, N-acetylimidazole, 2-amino benzimidazole, N-(3-aminopropyl)imidazole, 5-amino-2-(trifluoromethyl)benzimidazole, 4-zabenzimidazole, 4-aza-2-mercaptobenzimidazole, benzimidazole, 1-benzilimidazole, and 1-butylimidazole). The concentration of the compound containing an imidazole ring is able to be selected as appropriate. In view of obtaining sufficiently high buffer ability, the concentration thereof is suitably from 0.2 M to 3 M both inclusive, is more suitably from 0.2 M to 2.5 M both inclusive, and is much more suitably from 1 M to 2.5 M both inclusive. In the case where the concentration of the buffer substance contained in the electrolyte is sufficiently high from 0.2 M to 3 M both inclusive as described above, even if protons are increased or decreased in the electrode or in an enzyme immobilized film by enzyme reaction through protons or the like at the time of high output operation, sufficient buffer action is able to be obtained. Thus, shift of pH of the electrolyte around the enzyme from the optimum pH is able to be kept small sufficiently, and ability inherent in the enzyme is able to be sufficiently demonstrated. pKₐ of the buffer substance is generally from 5 to 9 both inclusive. Though pH of the electrolyte containing the buffer substance is suitably in the vicinity of 7, pH of the electrolyte containing the buffer substance may be generally any value out of a range from 1 to 14 both inclusive.

The buffer substance may contain a buffer substance other than the compound containing an imidazole ring according to needs. Specific examples thereof include dihydrogen phosphate ion (H₂PO₄⁻), 2-amino-2-hydroxymethyl-1,3-propanediol (abbreviated to tris), 2-(N-morpholino)ethane sulfonic acid (MES), cacodylic acid, carbonic acid (H₂CO₃), hydrogen citrate ion, N-(2-acetoamide)iminodiacetic acid (ADA), piperazine-N,N'-bis(2-ethane sulfonic acid) (PIPES), N-(2-acetoamide)-2-aminoethane sulfonic acid (ACES), 3-(N-morpholino)propanesulfonic acid (MOPS), N-2-hydroxyethyl pipezine-N'-2-ethane sulfonic acid (HEPES), N-2-hydroxyethyl piperazine-N'-3-propane sulfonic acid (HEPPS), N-[tris(hydroxymethyl)methyl]glycine (abbreviated to tricine), glycylglycine, and N,N-bis(2-hydroxyethyl) glycine (abbreviated to bicin).
Further, in view of retaining higher enzyme activity, a neutralizing agent, more specifically, for example, one or more acids selected from the group consisting of acetic acid (CH₃COOH), phosphoric acid (H₃PO₄), and sulfuric acid (H₂SO₄) are added in addition to the foregoing buffer substance, specially, the compound containing an imidazole ring. Further, in particular, in the case where a buffer solution in which the compound containing an imidazole ring is contained in the buffer substance, and to which one or more acids selected from the group consisting of acetic acid, phosphoric acid, and sulfuric acid are added is used, it is suitable that the viscosity of the buffer solution is sufficiently low for the following reason. That is, since excessively high viscosity of the buffer solution works against supplying a fuel and an electrolytic solution to the anode and the cathode, it is suitable that the viscosity of the buffer solution is sufficiently low to prevent such trouble. Specifically, the concentration of the buffer solution is suitably from 0.9 mPa·s to 2.0 mPa·s both inclusive. For example, in the case where the concentration of the buffer substance is from 0.2M to 2.5 M both inclusive, the viscosity of the buffer solution satisfies such conditions.

As an electrolyte, various materials may be used as long as a material does not have electron conductivity and is able to transport protons, and thus the electrolyte is selected according to needs. Specific examples of electrolyte include cellophane, a perfluorocarbon sulfonic acid (PFS) resin film, a copolymer film of a trifluorostyrene derivative, a polybenzimidazole film impregnated with phosphoric acid, an aromatic polyether ketone sulfonic acid film, PSSA-PVA (polystyrene sulfonic acid polyvinyl alcohol copolymer), PSSA-EVOH (polystyrene sulfonic acid ethylenevinyl alcohol copolymer), and a material composed of an ion exchange resin having a fluorine-containing carbon sulfonate group (for example, Nafion (product name, DuPont USA make)).

As the enzyme immobilized to one or both of the cathode and the anode, various enzymes may be used, and such enzyme is selected according to needs. Further, in addition to the enzyme, it is suitable that an electron mediator is immobilized to one or both of the cathode and the anode. According to needs, the buffer substance that contains the compound containing an imidazole ring may be immobilized to an immobilized film of the enzyme or the electron mediator.
Specifically, for example, in the case where a monomeric sugar such as glucose is used as a fuel, the enzyme immobilized to the anode contains an oxidase that promotes oxidation of the monomeric sugar and degrades the same, and generally contains a coenzyme oxidase that returns a coenzyme reduced by the oxidase to an oxidant in addition to the oxidase. By action of the coenzyme oxidase, electrons are generated when the coenzyme is returned to the oxidant, and the electrons are delivered from the coenzyme oxidase to the electrode through the electron mediator. As an oxidase, for example, NAD⁺ dependent glucose dehydrogenase (GDH) is used. As a coenzyme, for example, nicotine amide adenine dinucleotide (NAD⁺) is used. As a coenzyme oxidase, for example, diaphorase is used.

In the case where a polysaccharides is used as a fuel, in addition to the foregoing oxidase, the foregoing coenzyme oxidase, the foregoing coenzyme, and the foregoing electron mediator, a degradation enzyme that promotes degradation such as hydrolysis of the polysaccharide to generate a monomeric sugar such as glucose is also immobilized. In the specification, the polysaccharides are polysaccharides in the broad sense of the term, mean all carbohydrates from which a monomeric sugar with two or more molecules is generated by hydrolysis, and include oligosaccharide such as a disaccharide, a trisaccharide, and a tetrasaccharide. Specific examples of polysaccharides include starch, amylose, amylopectin, glycogen, cellulose, maltose, sucrose, and lactose. In such a polysaccharide, two or more monomeric sugars are bound. In any polysaccharide, glucose is contained as a monomeric sugar as a binding unit. Amylose and amylopectin are components contained in starch. Starch is a mixture of amylose and amylopectin. In the case where glucoamylase is used as a degradation enzyme of a polysaccharide and glucose dehydrogenase is used as an oxidase that degrades a monomeric sugar, if a substance contains a polysaccharide capable of being degraded to glucose by glucoamylase, power is able to be generated by using the substance as a fuel. Specific examples of such a polysaccharide include starch, amylose, amylopectin, glycogen, and maltose. Glucoamylase is a degradation enzyme that hydrolyzes α-glucan such as starch to generate glucose. Glucose dehydrogenase is an oxidase that oxidizes β-D-glucose to D-glucono-δ-lactone. It is suitable that as a structure in which the degradation enzyme degrading the polysaccharide is also immobilized onto the anode, a structure in which the polysaccharides to finally become a fuel is also immobilized onto the anode is adopted.

In the case where starch is used as a fuel, a gel solidified fuel obtained by gelatinizing starch is able to be used. In this case, it is suitable to use a method in which the gelatinized starch is contacted with the anode to which the enzyme or the like is immobilized, or a method in which the gelatinized starch is immobilized onto the anode together with the enzyme or the like. In the case where such an electrode is used, the starch concentration on the surface of the anode is able to be retained higher than that in a case of using starch dissolved in a solution, and degradation reaction by the enzyme becomes faster. Thus, output of the fuel cell is improved, handling the fuel is easier than in the case of using a solution, and the fuel supply system is able to be simplified. In addition, the fuel cell may be turned over. Thus, for example, in the case where the fuel cell is used for a mobile device, it becomes significantly advantageous.
In the case where methanol is used as a fuel, three-step oxidation process with the use of alcohol dehydrogenase (ADH) that acts on methanol as a catalyst and oxidizes methanol to formaldehyde, formaldehyde dehydrogenase (FalDH) that acts on formaldehyde and oxidizes formaldehyde to formic acid, and formic dehydrogenase (FateDH) that acts on formic acid and oxidizes formic acid to CO₂ is performed and degradation to CO₂ is accomplished In other words, three NADHs per one molecule of methanol are generated, and six electrons are generated.
In the case where ethanol is used as a fuel, two-step oxidation process with the use of alcohol dehydrogenase (ADH) that acts on ethanol as a catalyst and oxidizes ethanol to acetoaldehyde and aldehyde dehydrogenase (AlDH) that acts on acetoaldehyde and oxidizes acetoaldehyde to acetic acid is performed and degradation to acetic acid is accomplished. In other words, by two-stage oxidation reaction per one molecule of ethanol, four electrons in total are generated.
In ethanol, the method for degrading to CO₂ is able to be used as in methanol. In this case, after acetoaldehyde dehydrogenase (AalDH) acts on acetoaldehyde to obtain acetyl CoA, the resultant is delivered to a TCA cycle. In the TCA cycle, electrons are further generated.

As an electron mediator, any electron mediator may be used basically. However, a compound having a quinone skeleton, specially a compound having a naphthoquinone skeleton is suitably used. As the compound having a naphthoquinone skeleton, various naphthoquinone derivatives are able to be used. Specific examples of the naphthoquinone derivatives include 2-amino-1,4-naphthoquinone (ANQ), 2-amino-3-methyl-1,4-naphthoquinone (AMNQ), 2-methyl-1,4-naphthoquinone (VK3), and 2-amino-3-carboxy-1,4-naphthoquinone (ACNQ). As a compound having a quinone skeleton, in addition to the compound having a naphthoquinone skeleton, for example, anthraquinone and a derivative thereof are able to be used. In the electron mediator, according to needs, in addition to the compound having a quinone skeleton, one or more other compounds working as an electron mediator may be contained. As a solvent used in immobilizing the compound having a quinone skeleton, in particular, the compound having a naphthoquinone skeleton to the anode, acetone is suitably used. By using acetone as a solvent as described above, solubility of the compound having a quinone skeleton is able to be improved, and the compound having a quinone skeleton is able to be immobilized to the anode effectively. In the solvent, according to needs, one or more solvents other than acetone may be contained.

In one example, VK3 as an electron mediator, NADH as a coenzyme, glucose dehydrogenase as an oxidase, and diaphorase as a coenzyme oxidase are immobilized to the anode. Such VK3, NADH, glucose dehydrogenase, and diaphorase are suitably immobilized at a ratio of 1.0 (mol):0.33 to 1.0 (mol): (1.8 to 3.6)×10⁶ (U): (0.85 to 1.7)×10⁷ (U), where U (unit) is one index indicating enzyme activity, and represents a degree at which 1 µmol of substrate is reacted per one minute under certain temperature and certain pH.

Meanwhile, in the case where the enzyme is immobilized to the cathode, the enzyme typically contains an oxygen reduction enzyme. As the oxygen reduction enzyme, for example, bilirubin oxidase, laccase, ascorbate oxidase or the like is able to be used. Some oxygen reduction enzymes (multicopper oxidase) are illustrated in Table 1. In this case, the electron mediator is suitably also immobilized to the cathode in addition to the enzyme. As an electron mediator, for example, potassium hexacyanoferrate, potassium ferricyanide, potassium octacyanotungstate or the like is used. The electron mediator is suitably immobilized at a sufficiently high concentration, for example, at average 0.64×10⁻⁶ mol/mm² or more.

**[Table 1]**

| Name | Other name or the like | Origin | Function within organism | Molecular weight | Remarks |
|---|---|---|---|---|---|
| Plantlaccase | Polyphenol oxidase | Plant | Lignin formation Glycoprotein | 450 to 600 aa both inclusive | pH: about 5, Max pH: 8 Myrothecium |
| Fungal laccase | Same as above | Fungi | Pigment formation Lignin decomposition Glycoprotein | Same as above | 50 deg C |
| Fet3p | Ferroxidase | Saccharomyces cerevisiae | Iron metabolism Membrane protein Glycoprotein | 85 kDa | pH range: 2 to 9 both inclusive |
| Hephaestin | Same as above | Human | Same as above | 130 kDa | |
| Ceruloplasmin | Same as above | Same as above | Iron metabolism Glycoprotein | 130 to 135 kDa both inclusive | Optimum pH: 6.5 KmO₂: 10⁻² to 10⁻³ both inclusive |
| CueO (YacK) | cuiD, CopA | *E. coli* or the like | Copper homeostasis | 500 aa | |
| PcoA | | *E. coli* | Copper resistance | 565 aa | |
| CotA | Spore coat protein | Bacillus (spore forming bacterium) Gram-positive | Spore formation Mn(II) oxidation | 65 kDa | 80 deg C (2 to 4 hours both inclusive) |
| CumA | | Pseudomonas Gram-negative | | | |
| Ascorbate oxidase | | Plant (vegetable and fruit) | Ascorbic acid metabolism Glycoprotein | 140 kDa | Maximum temperature: 60 deg C 30 min |

As an immobilizer used for immobilizing the enzyme, the coenzyme, the electron mediator and the like to the anode or the cathode, various immobilizers are able to be used. As such an immobilizer, a polyion complex formed by using a polycation such as poly-L-lysine (PLL) or a salt thereof and a polyanion such as polyacrylic acid (for example, sodium polyacrylate (PAAcNa)) or a salt thereof is able to be used suitably. The enzyme, the coenzyme, the electron mediator and the like may be contained in the polyion complex. As an immobilizer, a material composed of poly-L-lysine and glutaraldehyde is able to be used as well.

In the case where the electron mediator is immobilized to the cathode and the anode of the fuel cell, since the electron mediator generally has low molecular weight, it is not always easy to perfectly inhibit elution and retain the state that the electron mediator is immobilized to the cathode and the anode for a long time. Thus, it is possible that the electron mediator used for the cathode is moved to the anode side, or on the contrary the electron mediator used for the anode is moved to the cathode side. In this case, it may lead to lowering of the output of the fuel cell and lowering of the electric capacity. To resolve the problem, it is effective to use an electrolyte having the same sign electric charge as that of an oxidant or a reductant of the electron mediator. Thereby, repulsion force works between the electric charge of the electrolyte and the electric charge of the oxidant or the reductant of the electron mediator. Accordingly, the electron mediator is hardly moved to the electrolyte side, and the electron mediator is effectively inhibited from being moved to the opposite side through the electrolyte. Typically, in the case where a polymer having the same sign electric charge as that of an oxidant or a reductant of the electron mediator such as a polyanion and a polycation is contained in the electrolyte, the electrolyte has the same sign electric charge as that of the oxidant or the reductant of the electron mediator. It is possible to use other method in order to obtain the state that the electrolyte has the same sign electric charge as that of the oxidant or the reductant of the electron mediator. Specifically, in the case where the oxidant or the reductant of the electron mediator used for one or both of the cathode and the anode has negative electric charge, a polymer having negative electric charge such as a polyanion is to be contained in the electrolyte. Meanwhile, in the case where the oxidant or the reductant of the electron mediator has positive electric charge, a polymer having positive electric charge such as a polycation is to be contained in the electrolyte. As a polyanion, Nafion (product name, DuPont USA make) as an ion exchange resin having a fluorine-containing carbon sulfonate group is able to be used. In addition, as the polyanion, for example, dichromate ion (Cr₂O₇²⁻), paramolybdenum acid ion ([Mo₇O₂₄]⁶⁻), polyacrylic acid (for example, sodium polyacrylate (PAAcNa)) and the like are able to be used. As a polycation, for example, poly-L-lysine (PLL) and the like are able to be used.

Meanwhile, the inventors of the present invention have found phenomenon that output of the fuel cell is able to be largely improved by immobilizing phosphatide such as dimyristoylphosphatidylcholine (DMPC) to the anode in addition to the enzyme and the electron mediator. In other words, the inventors of the present invention have found that the phosphatide functions as a high output agent. For the reason why high output is able to be realized by immobilizing the phosphatide, various studies have been made. In result, it was found that one of the reasons why sufficient high output was not able to be realized in the existing fuel cell was that the enzyme and the electron mediator immobilized to the anode were not uniformly mixed, and both the enzyme and the electron mediator were separated and in aggregation state. It resulted in a conclusion that by immobilizing the phosphatide, the enzyme and the electron mediator were able to be prevented from being separated and becoming in aggregation state, and the enzyme and the electron mediator were able to be uniformly mixed. Further, the reason why the enzyme and the electron mediator were able to be uniformly mixed by adding the phosphatide was studied. In result, significantly rare phenomenon that diffusion coefficient of the reductant of the electron mediator was largely increased by adding the phosphatide was found. In other words, they found a fact that the phosphatide functioned as an electron mediator diffusion promoter. In particular, such effect of immobilizing the phosphatide is significant in the case where the electron mediator is the compound having a quinone skeleton. It is able to obtain similar effect by using a derivative of the phosphatide or a polymer of the phosphatide or a derivative thereof instead of the phosphatide. In most generally speaking, the high output agent is an agent that improves reaction rate in the electrode to which the enzyme and the electron mediator are immobilized and is able to obtain high output. Further, in most generally speaking, the electron mediator diffusion promoter is a promoter capable of increasing the diffusion coefficient in the electron mediator in the electrode to which the enzyme and the electron mediator are immobilized, or a promoter retaining or increasing the concentration of the electron mediator in the vicinity of the electrode.

As a material of the cathode or the anode, a known material such as a carbon material is able to be used. In addition, a porous conductive material containing a skeleton made of a porous material and a material that has the carbon material as a main component and covers at least partial surface of the skeleton is able to be used. The porous conductive material is able to be obtained by coating at least partial surface of the skeleton made of the porous material with the material that has the carbon material as a main component. The porous material structuring the skeleton of the porous conductive material may be any material basically as long as the material is able to stably retain the skeleton even if the porosity ratio is high, and presence of its conductivity is not required. As a porous material, it is suitable to use a material having high porosity ratio and high conductivity. As a porous material having high porosity ratio and high conductivity, specifically, a metal material (a metal or an alloy), a carbon material in which the skeleton is intensified (fragility is resolved) and the like are able to be used. In the case where the metal material is used as a porous material, since state stability of the metal material varies according to usage environment such as pH of the solution and electric potential, various options are available. Specifically, examples of easily available metal material include a foamed metal or a foamed alloy of nickel, copper, silver, gold, nickel-chromium alloy, stainless steel and the like. As a porous material, in addition to the foregoing metal material or the carbon material, a resin material (for example, a sponge-like resin material) is able to be used. The porosity ratio and the pore diameter (minimum diameter of a pore) of the porous material are determined according to the porosity ratio and the pore diameter needed for the porous conductive material with the thickness of the material having the carbon material as a main component that covers the surface of the skeleton made of the porous material in mind. The pore diameter of the porous material is generally from 10 nm to 1 mm both inclusive, and is typically from 10 nm to 600 µm both inclusive. Meanwhile, as a material covering the surface of the skeleton, a material that has conductivity and is stable in envisaged working electric potential should be used. In the present invention, as such a material, a material having a carbon material as a main component is used. In general, many carbon materials have a wide electric potential window, and are chemically stable. Specifically, the material having the carbon material as a main component is categorized into a material composed of only the carbon material and a material containing the carbon material as a main component and containing a slightest amount of a side material selected according to characteristics needed for the porous conductive material and the like. Specific examples of the latter material include a material in which electric conductivity is improved by adding a high conductive material such as a metal to a carbon material and a material to which function other than conductivity such as giving surface water repellency by adding a polytetrafluoroethylene material or the like to the carbon material is given. Though the carbon material include various types, any carbon material may be used. In addition to carbon simple substance, a material obtained by adding other element to carbon may be used. As such a carbon material, in particular, a microscopic powder carbon material having high conductivity and high surface area is preferable. As the carbon material, specifically, a material having high conductivity such as KB (Ketjen black), a functional carbon material such as carbon nanotube and fullerene and the like are able to be used. As coating method of the material having the carbon material as a main component, any coating method may be used as long as the surface of the skeleton made of the porous material is able to be coated by using an appropriate binder according to needs. The pore diameter of the porous conductive material is selected as a size with which a solution containing a substrate or the like is able to easily come and go through the hole thereof. The pore diameter of the porous conductive material is generally from 9 nm to 1 mm both inclusive, is more generally from 1 µm to 1 mm both inclusive, and is much more generally from 1 to 600 µm both inclusive. In a state that at least partial surface of the skeleton made of the porous material is coated with the material having the carbon material as a main component, or in a state that at least partial surface of the skeleton made of the porous material is coated with the material having the carbon material as a main component, it is desirable that all pores are communicated with each other, or clogging by the material having the carbon material as a main component is not generated.

The entire structure of the fuel cell is selected according to needs. In the case where the fuel cell has, for example, a coin type structure or a button type structure, it is suitable that the cathode, the electrolyte, and the anode are contained in a space formed between the cathode current collector having a structure through which an oxidant is able to be permeated and the anode current collector having a structure through which a fuel is able to be permeated. In this case, it is typical that a space between an edge of one out of the cathode current collector and the anode current collector and the other out of the cathode current collector and the anode current collector is caulked with an insulative sealing member, and therefore the space in which the cathode, the electrolyte, and the anode are contained is formed. However, the structure is not limited thereto. The space in which the cathode, the electrolyte, and the anode are contained may be formed by other processing method according to needs. The cathode current collector and the anode current collector are electrically insulated from each other by an insulative sealing member. As the insulative sealing member, typically, gaskets made of various elastic bodies such as silicon rubber are used, but the material of the insulative sealing member is not limited thereto. The planar shape of the cathode current collector and the anode current collector is able to be selected according to needs. Examples of the planar shape include a circle, an oval, a rectangle, and a hexagon. Typically, the cathode current collector has one or a plurality of oxidant supply ports, and the anode current collector has one or a plurality of fuel supply ports, but the structures thereof are not limited. For example, it is possible that the oxidant supply port is not formed by using a material through which the oxidant is able to be permeated as a material of the cathode current collector. It is possible that the fuel supply port is not formed by using a material through which the fuel is able to be permeated as a material of the anode current collector. The anode current collector typically has a fuel retention section. The fuel retention section may be provided integrally with the anode current collector, or a removable fuel retention section may be provided with respect to the anode current collector. The fuel retention section typically has a sealing cover. In this case, it is possible that the cover is removed and the fuel is injected into the fuel retention section. It is also possible that the sealing cover is not used, and the fuel is injected from a side face or the like of the fuel retention section. In the case where the removable fuel retention section is provided with respect to the anode current collector, for example, as the fuel retention section, a fuel tank, a fuel cartridge or the like previously filled with the fuel may be attached. The fuel tank or the fuel cartridge may be disposable. However, in view of attaining effective usage of resources, the fuel tank or the fuel cartridge is preferably able to be filled with the fuel. Further, the used fuel tank or the used fuel cartridge may be changed to a fuel tank or a fuel cartridge filled with the fuel. Further, for example, the fuel cell is able to be continuously used by forming the fuel retention section in a state of a sealing container having a fuel supply port and an exhaust port, and continuously supplying the fuel from outside into the sealing container through the supply port. Alternatively, it is possible that the fuel retention section is not provided in the fuel cell, and the fuel cell is used in a state that the fuel cell is floated on a fuel contained in the open fuel tank with the anode side downward and the cathode side upward.

It is possible that the fuel cell has a structure in which the anode, the electrolyte, the cathode, and the cathode current collector having the structure through which the oxidant is able to be permeated are sequentially provided around a given central axis, and the anode current collector having the structure through which the fuel is able to be permeated is electrically connected to the anode. In the fuel cell, the anode may have a cylindrical cross sectional shape of a circle, an oval, a polygon or the like, or the anode may have a columnar cross sectional shape of a circle, an oval, a polygon or the like. In the case where the anode has a cylindrical shape, for example, the anode current collector may be provided on the inner circumferential side of the anode, may be provided between the anode and the electrolyte, may be provided in at least one end face of the anode, or may be provided in two or more sections thereof. Further, the anode may retain the fuel. For example, it is possible that the anode is formed from a porous material, and the anode also functions as a fuel retention section. Alternatively, a columnar fuel retention section may be provided on a given central axis. For example, in the case where the anode current collector is provided on the inner circumferential face side of the anode, the fuel retention section may be a space itself surrounded by the anode current collector, or the fuel retention section may be a container such as a fuel tank and a fuel cartridge provided separately from the anode current collector in the space. Further, the container may be removable, or may be fixed. The fuel retention section is, for example, in a state of a column, an elliptical column, a multangular column such as a quadrangular column and a hexangular column and the like, but the shape thereof is not limited thereto. The electrolyte may be formed as a pouch-like container to wrap around the anode and the anode current collector as a whole. Thereby, in the case where the fuel retention section is fully filled with the fuel, the fuel is able to be contacted with the entire anode. It is possible that at least a section sandwiched between the cathode and the anode out of the container is formed from the electrolyte, and the other sections are formed from a material different from the electrolyte. The fuel cell is able to be continuously used by forming the container in a state of a sealing container having a fuel supply port and an exhaust port, and continuously supplying the fuel from outside into the container through the supply port. As an anode, in order to sufficiently store the fuel inside, an anode having large void ratio is preferable, and for example, an anode having void ratio of 60% or more is preferable.

As a cathode and an anode, a pellet electrode is also able to be used. The pellet electrode is able to be formed, for example, as follows. That is, a carbon material, a binder according to needs, the foregoing enzyme powder, coenzyme powder, electron mediator powder, polymer powder for immobilization and the like are mixed in an agate mortar or the like. The resultant mixture is dried as appropriate, and is pressed into a given shape. As a carbon material, in particular, a minute powder carbon material having high conductivity and high surface area is preferable. Specifically, a material having high conductivity such as KB (Ketjen black), a functional carbon material such as carbon nanotube and fullerene and the like are used. As a binder, for example, polyvinylidene fluoride is used. An enzyme solution, a coenzyme solution, an electron mediator solution, and a polymer solution may be used instead of the enzyme powder, the coenzyme powder, the electron mediator powder, and the polymer powder for immobilization. The thickness of the pellet electrode (electrode thickness) is determined according to needs, for example, is about 50 µm. For example, in the case where a coin type fuel cell is manufactured, the pellet electrode is able to be formed by pressing the foregoing materials for forming the pellet electrode into a circular shape by a tablet manufacturing machine. For example, a diameter of the circular pellet electrode is 15 mm, but the diameter is not limited thereto but is determined according to needs. In the case where the pellet electrode is formed, for obtaining a desired electrode thickness, for example, a carbon amount proportion out of the materials for forming the pellet electrode, pressure and the like are controlled. In inserting the cathode or the anode in a coin-type battery can, for example, it is preferable to insert a metal mesh spacer between the cathode/the anode and the battery can to obtain electric contact thereof.

As a method of manufacturing the pellet electrode, in addition to the foregoing method, the following method may be used. That is, a current collector or the like is coated with a carbon material, a binder according to needs, and a mixed solution (an aqueous solution or an organic solvent mixed solution) of enzyme immobilization components (an enzyme, a coenzyme, an electron mediator, a polymer and the like) as appropriate, the resultant is dried, and the entire body is pressed. After that, the pressed resultant is cut into a desired electrode size, and therefore the pellet electrode is able to be manufactured.
The fuel cell is able to be used for all devices necessitating electric power without relation to device size. The fuel cell is able to be used, for example, for an electronic device, a movable body (a car, a two-wheel bicycle, an aircraft, a rocket, a spaceship or the like), a motor, a construction machine, a machine tool, a power generation system, a cogeneration system and the like. According to the purpose of the fuel cell and the like, the output, the size, the shape, the fuel type and the like are determined.

A second aspect in accordance with the present invention provides an electronic device having one or a plurality of fuel cells, wherein one or more of the fuel cells has a structure in which a cathode and an anode are opposed to each other with an electrolyte containing a buffer substance in between, an enzyme is immobilized to one or both of the cathode and the anode, and a compound containing an imidazole ring is contained in the buffer substance.
The electronic device may be any type basically, and includes both a portable electronic device and a stationary electronic device. Specific examples thereof include a mobile phone, a mobile device, a robot, a personal computer, a game machine, an on-vehicle device, a home electric appliance, and an engineering product.
In the second aspect in accordance with the present invention, the descriptions given for the first aspect in accordance with the present invention are affected.

A third aspect in accordance with the present invention provides a fuel cell having a structure in which a cathode and an anode are opposed to each other with an electrolyte containing a buffer substance in between, wherein an enzyme is immobilized to one or both of the cathode and the anode, and at least one selected from the group consisting of 2-aminoethanol, triethanol amine, TES, and BES is contained in the buffer substance.
TES represents N-Tris(hydroxymethyl)methyl-2-aminoethanesulfonic acid, and BES represents N,N-Bis(2-hydroxyethyl)-2-aminoethanesulfonic acid. In the third aspect in accordance with the present invention, according to needs, the compound containing an imidazole ring or other buffer substance may be contained in the buffer substance, or one or more acids selected from the group consisting of acetic acid, phosphoric acid, and sulfuric acid may be added.
In the third aspect in accordance with the present invention, the descriptions given for the first aspect and the second aspect in accordance with the present invention are affected, as long as the contents do not work against the characteristics. Further, in the third aspect in accordance with the present invention, advantages similar to those of the first aspect in accordance with the present invention are able to be obtained.
A fourth aspect in accordance with the present invention provides a buffer solution for a fuel cell used for a fuel cell which has a structure in which a cathode and an anode are opposed to each other with an electrolyte containing a buffer substance in between, and in which an enzyme is immobilized to one or both of the cathode and the anode, wherein a compound containing an imidazole ring is contained in the buffer substance, and one or more acids selected from the group consisting of acetic acid, phosphoric acid, and sulfuric acid are added.
In the fourth aspect in accordance with the present invention, the descriptions given for the first aspect in accordance with the present invention are affected, as long as the contents do not work against the characteristics.

In the aspects of the present invention structured as above, since the compound having an imidazole ring is contained in the buffer substance contained in the electrolyte, sufficient buffer ability is able to be obtained. Thus, even if protons are increased or decreased in the proton electrode or in the enzyme immobilized film by enzyme reaction through protons at the time of high output operation of the fuel cell, sufficient buffer ability is able to be obtained. In result, shift of pH of the electrolyte around the enzyme from the optimum pH is able to be kept small sufficiently. Further, by adding one or more acids selected from the group consisting of acetic acid, phosphoric acid, and sulfuric acid to the buffer substance, higher enzyme activity is able to be retained. Therefore, electrode reaction by the enzyme, the coenzyme, the electron mediator and the like is able to be efficiently and constantly initiated.

According to the aspects of the present invention, sufficient buffer ability is able to be obtained even at the time of high output operation, and ability inherent in the enzyme is able to be sufficiently demonstrated. Thereby, a fuel cell having superior performance is able to be obtained. In addition, by using the superior fuel cell, highly efficient electronic devices and the like are able to be realized.

### Brief Description of Drawings

[FIG. 1] FIG. 1 is a schematic diagram illustrating a bio-fuel cell according to a first embodiment of the invention.
[FIG. 2] FIG. 2 is a schematic diagram schematically illustrating details of a structure of an anode of the bio-fuel cell according to the first embodiment, an example of an enzyme group immobilized to the anode, and electron delivery and receipt reaction by the enzyme group.
[FIG. 3] FIG. 3 is a schematic diagram illustrating results of chronoamperometry performed for evaluating the bio-fuel cell according to the first embodiment.
[FIG. 4] FIG. 4 is a schematic diagram illustrating relation between buffer solution concentration and obtained current density that are obtained by the results of the chronoamperometry performed for evaluating the bio-fuel cell according to the first embodiment.
[FIG. 5] FIG. 5 is a schematic diagram illustrating a measurement system used for the chronoamperometry measurement illustrated in FIG. 3.
[FIG. 6] FIG. 6 is a schematic diagram illustrating results of cyclic voltammetry performed for evaluating the bio-fuel cell according to the first embodiment.
[FIG. 7] FIG. 7 is a schematic diagram illustrating a measurement system used for the cyclic voltammetry measurement illustrated in FIG. 6.
[FIG. 8] FIG. 8 is a schematic diagram illustrating results of chronoamperometry performed by using a buffer solution containing imidazole and an NaH₂PO₄ buffer solution in the bio-fuel cell according to the first embodiment.
[FIG. 9] FIG. 9 is a schematic diagram for explaining mechanism in which a large current is constantly able to be obtained in the case where the buffer solution containing imidazole is used in the bio-fuel cell according to the first embodiment.
[FIG. 10] FIG. 10 is a schematic diagram for explaining mechanism in which a current is decreased in the case where the NaH₂PO₄ buffer solution is used in the bio-fuel cell according to the first embodiment.
[FIG. 11] FIG. 11 is a schematic diagram illustrating relation between buffer solution concentration and current density in the case where various buffer solutions are used in the bio-fuel cell according to the first embodiment.
[FIG. 12] FIG. 12 is a schematic diagram illustrating relation between buffer solution concentration and current density in the case where various buffer solutions are used in the bio-fuel cell according to the first embodiment.
[FIG. 13] FIG. 13 is a schematic diagram illustrating relation between molecular weight of a buffer substance of a buffer solution and current density in the case where various buffer solutions are used in the bio-fuel cell according to the first embodiment.
[FIG. 14] FIG. 14 is a schematic diagram illustrating relation between pKₐ of a buffer solution and current density in the case where the various buffer solutions are used in the bio-fuel cell according to the first embodiment.
[FIG. 15] FIG. 15 is a schematic diagram illustrating relation between buffer solution concentration and buffer solution viscosity in the case where various buffer solutions are used in the bio-fuel cell according to the first embodiment.
[FIG. 16] FIG. 16 is a schematic diagram illustrating relation between buffer solution viscosity and an obtained current in the case where an imidazole/hydrochloric acid buffer solution is used in the bio-fuel cell according to the first embodiment.
[FIG. 17] FIG. 17 is a schematic diagram illustrating relation between glucose concentration and buffer solution viscosity in the case where the imidazole/hydrochloric acid buffer solution is used in the bio-fuel cell according to the first embodiment.
[FIG. 18] FIG. 18 is a schematic diagram illustrating relation between buffer solution concentration and buffer solution viscosity/molecule diffusion coefficient in the case where the buffer solution containing imidazole and the NaH₂PO₄ buffer solution are used in the bio-fuel cell according to the first embodiment.
[FIGS. 19] FIGS. 19 are schematic diagrams illustrating a specific structural example of the bio-fuel cell according to the first embodiment.
[FIG. 20] FIG. 20 is a schematic diagram illustrating measurement results of output of the bio-fuel cell used for evaluation in the first embodiment.
[FIGS. 21] FIGS. 21 are schematic diagrams illustrating results of cyclic voltammetry performed for validating transmission preventive effect of an electron mediator in a bio-fuel cell according to a second embodiment of the present invention.
[FIG. 22] FIG. 22 is a schematic diagram illustrating a measurement system used for the cyclic voltammetry performed for validating transmission preventive effect of the electron mediator in the bio-fuel cell according to the second embodiment.
[FIG. 23] FIG. 23 is a schematic diagram illustrating results of the cyclic voltammetry performed for validating the transmission preventive effect of the electron mediator in the bio-fuel cell according to the second embodiment.
[FIG. 24] FIG. 24 is a schematic diagram illustrating results of the cyclic voltammetry performed for validating the transmission preventive effect of the electron mediator in the bio-fuel cell according to the second embodiment.
[FIGS. 25] FIGS. 25 are a top view, a cross sectional view, and a rear face view of a bio-fuel cell according to a third embodiment.
[FIG. 26] FIG. 26 is an exploded perspective view illustrating the bio-fuel cell according to the third embodiment.
[FIGS. 27] FIGS. 27 are schematic diagrams for explaining a method of manufacturing the bio-fuel cell according to the third embodiment.
[FIG. 28] FIG. 28 is a schematic diagram for explaining a first example of a usage method of the bio-fuel cell according to the third embodiment.
[FIG. 29] FIG. 29 is a schematic diagram for explaining a second example of a usage method of the bio-fuel cell according to the third embodiment.
[FIG. 30] FIG. 30 is a schematic diagram for explaining a third example of a usage method of the bio-fuel cell according to the third embodiment.
[FIG. 31] FIG. 31 is a schematic diagram illustrating a bio-fuel cell according to a fourth embodiment and a usage method thereof.
[FIGS. 32] FIGS. 32 are an elevation view and a vertical cross sectional view illustrating a bio-fuel cell according to a fifth embodiment.
[FIG. 33] FIG. 33 is an exploded perspective view illustrating the bio-fuel cell according to the fifth embodiment.
[FIGS. 34] FIGS. 34 are a schematic diagram and a cross sectional view for explaining a structure of a porous conductive material used for an electrode material of an anode in a bio-fuel cell according to a sixth embodiment.
[FIGS. 35] FIGS. 35 are schematic diagrams for explaining a method of manufacturing the porous conductive material used for the electrode material of the anode in the bio-fuel cell according to the sixth embodiment.
[FIG. 36] FIG. 36 is a schematic diagram illustrating results of cyclic voltammetry performed by using one type or many types of electron mediators in the bio-fuel cell.
[FIG. 37] FIG. 37 is a schematic diagram illustrating results of cyclic voltammetry performed by using one type or many types of electron mediators in the bio-fuel cell.
[FIG. 38] FIG. 38 is a schematic diagram illustrating results of cyclic voltammetry performed by using one type or many types of electron mediators in the bio-fuel cell.

### Description of Embodiments

Embodiments of the present invention will be hereinafter described. The description will be given in the following order.
1. First embodiment (bio-fuel cell)
2. Second embodiment (bio-fuel cell)
3. Third embodiment (bio-fuel cell and method of manufacturing the same)
4. Fourth embodiment (bio-fuel cell)
5. Fifth embodiment (bio-fuel cell)
6. Sixth embodiment (bio-fuel cell)
7. Seventh embodiment (bio-fuel cell)

### <1. First embodiment>

### [Bio-fuel cell]

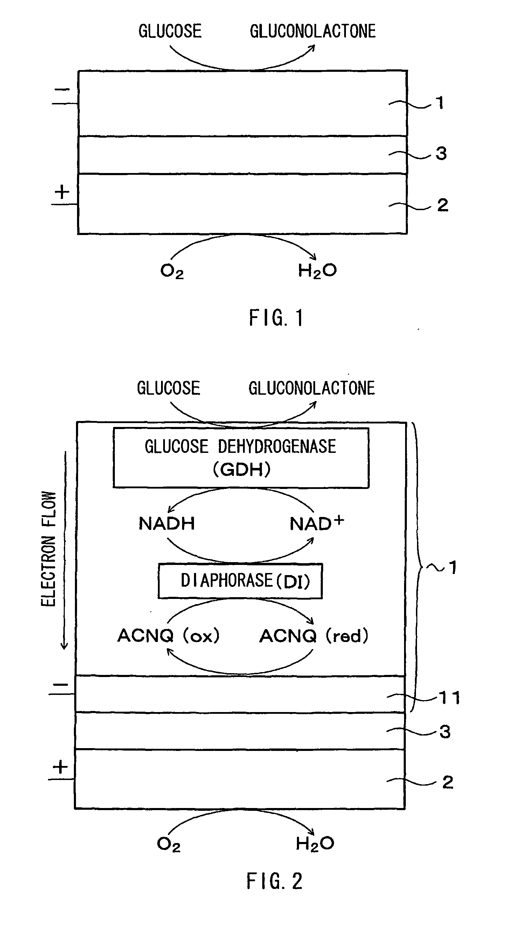

FIG. 1 schematically illustrates a bio-fuel cell according to a first embodiment of the present invention. In the bio-fuel cell, glucose is used as a fuel. FIG. 2 schematically illustrates details of a structure of an anode of the bio-fuel cell, an example of an enzyme group immobilized to the anode, and electron delivery and receipt reaction by the enzyme group.
The bio-fuel cell has a structure in which an anode 1 and a cathode 2 are opposed to each other with an electrolyte layer 3 that conducts only protons in between. In the anode 1, glucose supplied as a fuel is degraded by an enzyme, electrons are extracted, and the protons (H⁺) are generated. In the cathode 2, water is generated from the protons transported from the anode 1 through the electrolyte layer 3, the electrons sent from the anode 1 through an external circuit, and, for example, oxygen in the air.

In the anode 1, the enzyme related to degrading glucose, a coenzyme, a coenzyme oxidase, an electron mediator and the like are immobilized onto an electrode 11 (refer to FIG. 2) made of, for example, porous carbon with the use of an immobilizer composed of, for example, a polymer. From the coenzyme, a reductant is generated in association with oxidation reaction in degradation process of glucose. For example, the coenzyme is NAD⁺, NADP⁺ or the like. The coenzyme oxidase is a coenzyme oxidase that oxidizes the reductant of the coenzyme (for example, NADH or NADPH). For example, the coenzyme oxidase is diaphorase. The electron mediator receives electrons generated in association with oxidization of the coenzyme from the coenzyme oxidase, and delivers the electrons to the electrode 11.

As the coenzyme related to degradation of glucose, for example, glucose dehydrogenase (GDH) is able to be used. In the case where such an oxidase exists, for example, β-D-glucose is able to be oxidized to D-glucono-δ-lactone.
Further, in the case where two enzymes that are gluconokinase and phosphogluconate dehydrogenase (PhGDH) exist, such D-glucono-δ-lactone is able to be degraded to 2-keto-6-phospho-D-gluconate. In other words, D-glucono-δ-lactone becomes D-gluconate by hydrolysis. In the case where adenosine triphosphate (ATP) is hydrolyzed into adenosine diphosphate (ADP) and phosphoric acid under existence of gluconokinase, D-gluconate is phosphorylated and becomes 6-phospho-D-gluconate. Such 6-phospho-D-gluconate is oxidized to 2-keto-6-phospho-D-gluconate by action of oxidase PhGDH.

Further, in addition to the foregoing degradation process, glucose is able to be degraded to CO₂ by using sugar metabolism. The degradation process using the sugar metabolism is roughly divided into degradation of glucose by glycolytic system, generation of pyruvic acid, and TCA cycle, which are widely known reaction systems.
Oxidation reaction in degradation process of a monomeric sugar is initiated along with reduction reaction of a coenzyme. The coenzyme is almost determined by an action enzyme. In the case of GDH, NAD⁺ is used as a coenzyme. In other words, in the case where β-D-glucose is oxidized to D-glucono-δ-lactone by action of GDH, NAD⁺ is reduced to NADH and H⁺ is generated.

The generated NADH is immediately oxidized to NAD⁺ under existence of diaphorase (DI), and two electrons and H⁺ are generated. Thus, two electrons and two H⁺ are generated by one stage oxidation reaction per one molecule of glucose. In two stage oxidation reaction, four electrons and four H⁺ are generated in total.
The electrons generated in the foregoing process are delivered from diaphorase to the electrode 11 through the electron mediator. H⁺ are transported to the cathode 2 through the electrolyte layer 3.

The electron mediator receives/delivers electrons from/to the electrode 11. The output voltage of the fuel cell depends on the redox electric potential of the electron mediator. In other words, to obtain a higher output voltage, it is preferable to select an electron mediator having more negative electric potential on the anode 1 side. In selecting the electron mediator, it is necessary to consider reaction affinity of the electron mediator to the enzyme, electron exchange rate with respect to the electrode 11, structural stability with respect to an inhibitory agent (light, oxygen or the like) and the like. In view of the foregoing facts, as an electron mediator acting on the anode 1, 2-amino-3-carboxy-1,4-naphthoquinone (ACNQ), 2-methyl-1,4- naphthoquinone (VK3), 2-amino-1,4-naphthoquinone (ANQ) and the like are suitable. In addition, for example, a compound having a quinone skeleton, a metal complex of osmium (Os), ruthenium (Ru), iron (Fe), cobalt (Co) or the like, a viologen compound such as benzyl viologen and the like are able to be used as an electron mediator. Further, a compound having a nicotine amide structure, a compound having a riboflavin structure, a compound having a nucleotide-phosphoric acid structure and the like are able to be used as an electron mediator.

The electrolyte layer 3 is a proton conductor that transports H⁺ generated in the anode 1 to the cathode 2. The electrolyte layer 3 is made of a material that does not have electron conductivity and is able to transport H⁺. As the electrolyte layer 3, for example, one selected as appropriate from the group consisting of the above-mentioned substances is able to be used. In this case, in the electrolyte layer 3, a buffer solution containing a compound having an imidazole ring as a buffer substance is contained. As the compound having an imidazole ring, one from the group consisting of the above-mentioned substances such as imidazole is able to be selected as appropriate. The concentration of the compound having an imidazole ring as a buffer substance is selected according to needs. The concentration thereof is suitably from 0.2 M to 3 M both inclusive. Thereby, high buffer ability is able to be obtained. In addition, even at the time of high output operation of the fuel cell, ability inherent in the enzyme is able to be sufficiently demonstrated. Both excessively large ion intensity (I. S.) and excessively small ion intensity adversely affect enzyme activity. Considering electrochemical responsibility, moderate ion intensity such as about 0.3 is preferable. However, each optimum pH value and each optimum ion intensity value exist for each enzyme used, and thus the values are not limited to the foregoing values.

The foregoing enzyme, the foregoing coenzyme, and the foregoing electron mediator are preferably immobilized onto the electrode 11 by using an immobilizer in order to effectively capture enzyme reaction phenomenon initiated in the vicinity of the electrode as an electric signal. Further, it is able to stabilize enzyme reaction system of the anode 1 by immobilizing the enzyme degrading the fuel and the coenzyme onto the electrode 11 as well. As such an immobilizer, for example, a combination of glutaraldehyde (GA) and poly-L-lysine (PLL) and a polyion complex obtained by combining sodium polyacrylate (PAAcNa) and poly-L-lysine (PLL) may be used. As an immobilizer, the foregoing substances may be used singly, or other polymer may be used. Of the foregoing, in the case where an immobilizer obtained by combining glutaraldehyde and poly-L-lysine is used, each enzyme immobilization ability is able to be largely improved, and superior enzyme immobilization ability is able to be obtained as an entire immobilizer. In this case, for the composition ratio between glutaraldehyde and poly-L-lysine, the optimum value varies according to the enzyme to be immobilized and the substrate of the enzyme. However, in general, a given composition ratio is tolerable. Specific examples thereof include a case in which glutaraldehyde aqueous solution (0.125%) and poly-L-lysine aqueous solution (1%) are used, and the ratio thereof is, for example, 1:1, 1:2, or 2:1.

FIG. 2 illustrates a case in which the enzyme related to degradation of glucose is glucose dehydrogenase (GDH), the coenzyme from which a reductant is generated in association with oxidation reaction in degradation process of glucose is NAD⁺, the coenzyme oxidase that oxidizes NADH as a reductant of the coenzyme is diaphorase (DI), the electron mediator that receives electrons generated in association with oxidization of the coenzyme from the coenzyme oxidase and that delivers the electrons to the electrode 11 is ACNQ as an example.

In the cathode 2, the oxygen reduction enzyme and the electron mediator that receives/delivers electrons from/to the electrode are immobilized onto the electrode made of, for example, a porous carbon. As an oxygen reduction enzyme, for example, bilirubin oxidase (BOD), laccase, ascorbate oxidase or the like is able to be used. As an electron mediator, for example, hexacyanoferrate ion generated by electrolytic dissociation of potassium hexacyanoferrate is able to be used. The electron mediator is suitably immobilized at sufficiently high concentration such as average 0.64×10⁻⁶ mol/mm² or more.

In the cathode 2, under existence of the oxygen reduction enzyme, oxygen in the air is reduced by H⁺ from the electrolyte layer 3 and the electrons from the anode 1 and water is generated.
In the fuel cell structured as above, in the case where glucose is supplied to the anode 1 side, such glucose is degraded by a degradation enzyme containing the oxidase. Since the oxidase is related to such degradation process of the monomeric sugar, electrons and H⁺are able to be generated on the anode 1 side, and a current is able to be generated between the anode 1 and the cathode 2.

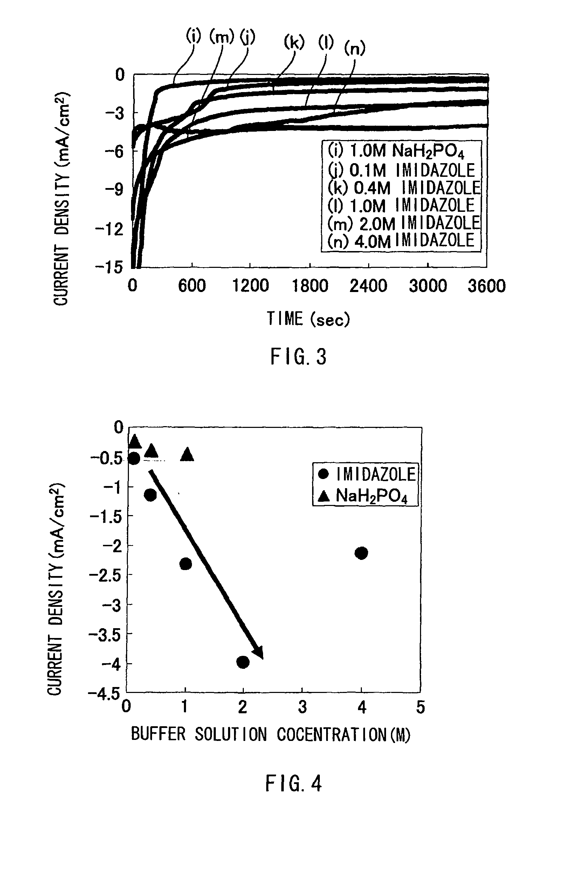

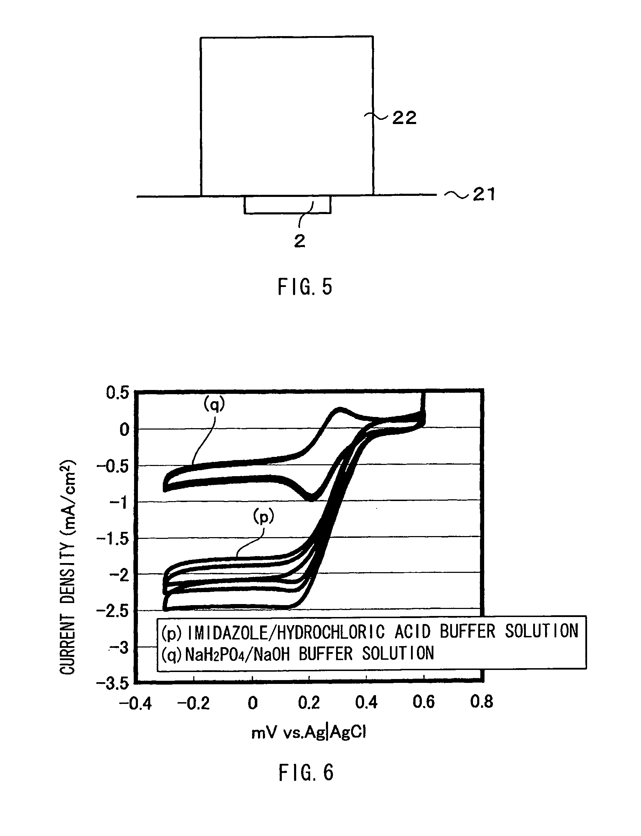

Next, a description will be given of effects of improving current value retention in the case where BOD as an oxygen reduction enzyme was immobilized to the cathode 2, and a solution obtained by mixing imidazole and hydrochloric acid and adjusting pH value to pH 7 was used as a buffer solution. As BOD, a product purchased from Amano Enzyme Inc. was used. Table 1 and FIG. 3 illustrate results of chronoamperometry measured by changing the concentration of imidazole in this case. Further, FIG. 4 illustrates buffer solution concentration (concentration of the buffer substance in the buffer solution) dependence of a current value (current density value after 3600 sec in Table 1 and FIG. 3). For comparison, Table 1 and FIG. 4 also illustrate results in a case that 1.0 M of NaH₂PO₄/NaOH buffer solution (pH7) was used as a buffer solution. As illustrated in FIG. 5, the measurement was performed in a state that a film-like cellophane 21 was laid on the cathode 2, and a buffer solution 22 was contacted with the cellophane 21. As the cathode 2, an enzyme/electron mediator-immobilized electrode formed as below was used. First, as porous carbon, commercially available carbon felt (BO050, TORAY make) was used, and the carbon felt was cut into a 1-cm-square piece. Next, the foregoing carbon felt was sequentially transfused with 80 µl of hexacyanoferrate ion (100 mM), 80 µl of poly-L-lysine (1 wt%), and 80 µl of a BOD solution (50 mg/ml), and the resultant was dried. Thereby, the enzyme/electron mediator-immobilized electrode was obtained. Two pieces of the enzyme/electron mediator-immobilized electrodes formed as above were layered, and the resultant was used as the cathode 2.

**[Table 2]**

| Current density (mA/cm²) | | | | | | |
|---|---|---|---|---|---|---|
| | 1 sec | 180 sec | 300 sec | 600 sec | 1800 sec | 3600 sec |
| 1.0 M NaH₂PO₄ | -17.22 | -3.11 | -1.10 | -0.73 | -0.41 | -0.34 |
| 0.1 M imidazole | -5.64 | -3.98 | -3.71 | -2.98 | -0.70 | -0.54 |
| 0.4 M imidazole | -11.18 | -6.37 | -4.69 | -2.48 | -1.35 | -1.16 |
| 1.0 M imidazole | -15.59 | -8.44 | -5.81 | -3.86 | -2.60 | -2.32 |
| 2.0 M imidazole | -25.10 | -7.39 | -5.88 | -5.01 | -4.20 | -3.99 |
| 4.0 M imidazole | -5.08 | -3.90 | -4.19 | -4.53 | -3.47 | -2.13 |

As evidenced by Table 2 and FIG. 3, in the case where the concentration of NaH₂PO₄ was 1.0 M, though the initial current was high, the current was largely decreased after 3600 seconds. Meanwhile, in particular, in the case where the concentration of imidazole was 0.4 M, 1.0 M, and 2.0 M, the current was hardly decreased even after 3600 seconds. As evidenced by FIG. 4, in the case where the concentration of imidazole was from 0.2 to 2.5 M both inclusive, the current value was linearly increased with respect to the concentration. Further, though both the NaH₂PO₄/NaOH buffer solution and the imidazole/hydrochloric acid buffer solution had pKₐ in the vicinity of 7 and almost the same oxygen solubility, in the case where imidazole existed in the buffer solution having the same concentration, a large oxygen reduction current was obtained.

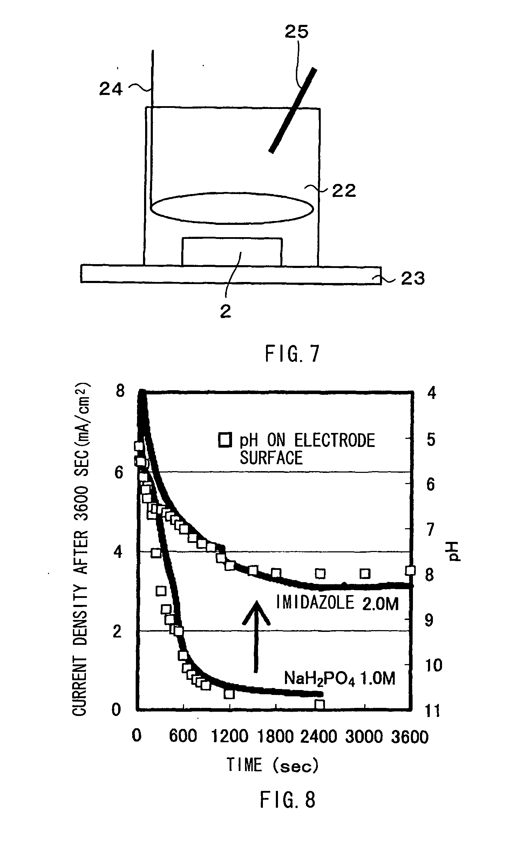

After chronoamperometry was performed for 3600 seconds as described above, cyclic voltammetry (CV) between electric potential -0.3 and electric potential +0.6 V was performed. The result is illustrated in FIG. 6. However, as illustrated in FIG. 7, the measurement was performed in a state that the cathode 2 composed of an enzyme/electron mediator-immobilized electrode similar to the foregoing enzyme/electron mediator-immobilized electrode was used as a working electrode, which was laid on an air permeable PTFE (polytetrafluoroethylene) membrane 23, and the buffer solution 22 was contacted with the cathode 2. A counter electrode 24 and a reference electrode 25 were soaked into the buffer solution 22. An electrochemical measurement equipment (not illustrated) was connected to the cathode 2 as a working electrode, the counter electrode 24, and the reference electrode 25. As the counter electrode 24, Pt line was used. As the reference electrode 25, Ag|AgCl was used. Measurement was performed under atmosphere pressure, and the measurement temperature was 25 deg C. As the buffer solution 22, two types of imidazole/hydrochloric acid buffer solution (pH7 and 1.0 M) and NaH₂PO₄/NaOH buffer solution (pH7 and 1.0 M) were used.
From FIG. 6, it was found that in the case where the imidazole/hydrochloric acid buffer solution (pH7 and 1.0 M) was used as the buffer solution 22, significantly favorable CV characteristics were able to be obtained.
Accordingly, it was found that even if the measurement system was changed, the imidazole buffer solution had superiority.

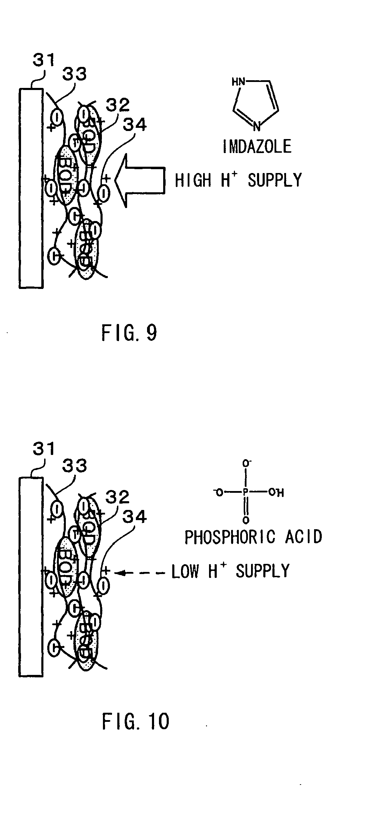

FIG. 8 illustrates results of chronoamperometry performed in a manner similar to the above-mentioned method by immobilizing BOD to the cathode 2 and using 2.0 M of imidazole/hydrochloric acid buffer solution and 1.0 M of NaH₂PO₄/NaOH buffer solution together with measurement result of pH on the electrode surface in the chronoamperometry. pKₐ of the imidazole/hydrochloric acid buffer solution was 6.95, the conductivity was 52.4 mS/cm, the oxygen solubility was 0.25 mM, and pH was 7. Further, pKₐ of the NaH₂PO₄/NaOH buffer solution was 6.82 (H₂PO₄⁻), the conductivity was 51.2 mS/cm, the oxygen solubility was 0.25 mM, and pH was 7. As evidenced by FIG. 8, in the case where 2.0 M of imidazole/hydrochloric acid buffer solution was used, the current density about 15 times the current density in the case of using 1.0 M of NaH₂PO₄/NaOH buffer solution was able to be obtained. Further, from FIG. 8, it was found that current change approximately corresponded with pH change on the electrode surface. For the reason why these results were able to be obtained, a description will be given with reference to FIG. 9 and FIG. 10.

FIG. 9 and FIG. 10 illustrate a state that a BOD 32 and an electron mediator 34 are immobilized to an electrode 31 by an immobilizer 33 such as a polyion complex. As illustrated in FIG. 9, in the case where 2.0 M of imidazole/hydrochloric acid buffer solution was used, sufficiently many protons (H⁺) were supplied and therefore high buffer ability was obtained and pH was stabilized, and accordingly high current density was constantly obtained. Meanwhile, as illustrated in FIG. 10, in the case where 1.0 M of NaH₂PO₄/NaOH buffer solution was used, since supply amount of H⁺ was small, buffer ability was not sufficient. Thus, since pH was largely increased, current density was decreased.

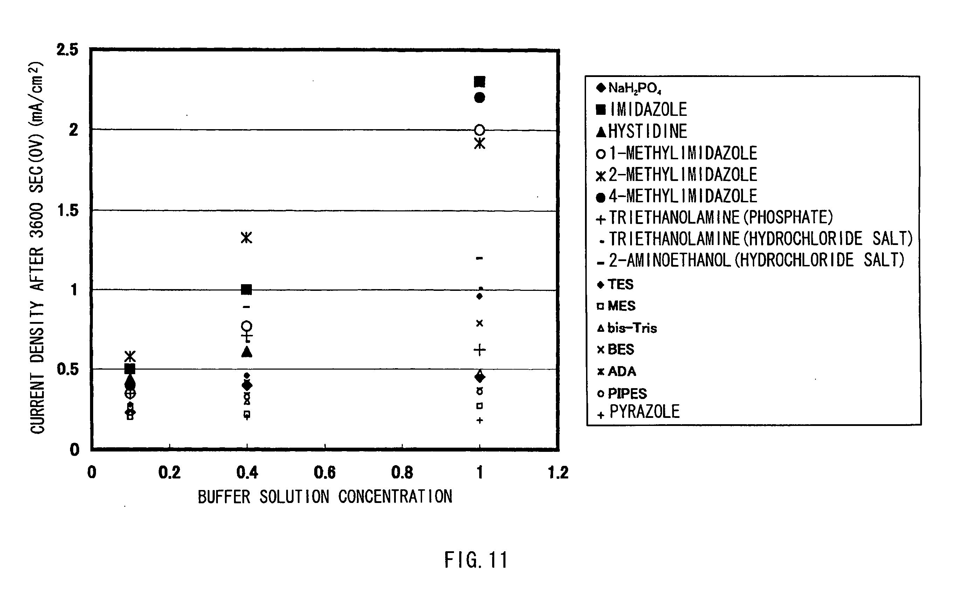

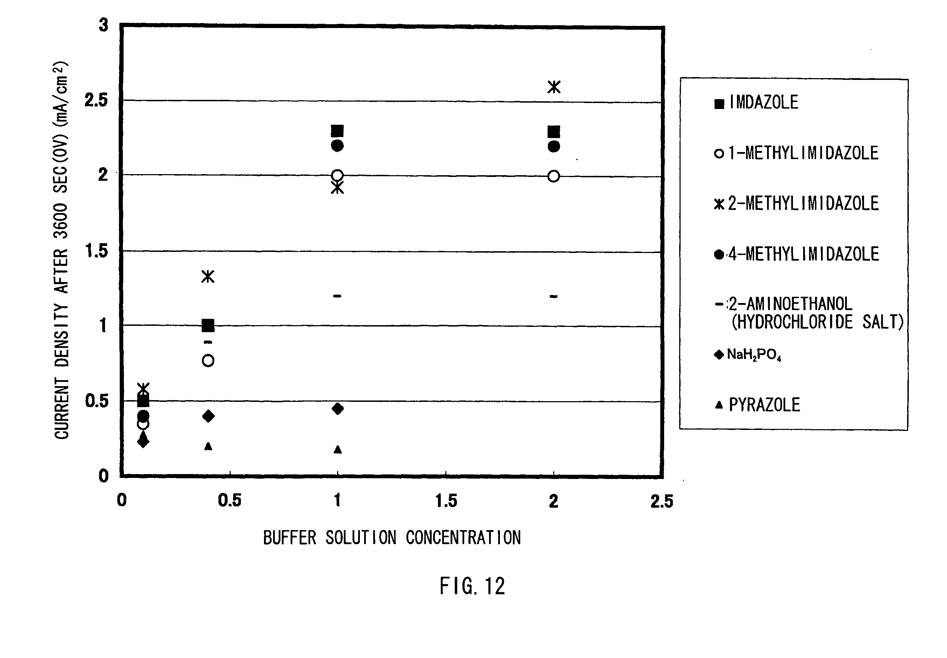

FIG. 11 and FIG. 12 illustrate change of current density after 3600 seconds (1 hour) in the case of using various buffer solutions with respect to buffer solution concentration. As evidenced by FIG. 11 and FIG. 12, in the case where a buffer solution containing a compound having an imidazole ring was used, higher current density was able to be obtained as a whole than that in a case of using other buffer solution such as a buffer solution containing NaH₂PO₄, and in particular, such tendency was significant as the buffer solution concentration was higher. Further, from FIG. 11 and FIG. 12, it was found that in the case where a buffer solution containing 2-aminoethanol, triethanol amine, TES, or BES as a buffer substance was used, high current density was obtained as well, and in particular, such tendency was significant as the buffer solution concentration was higher.

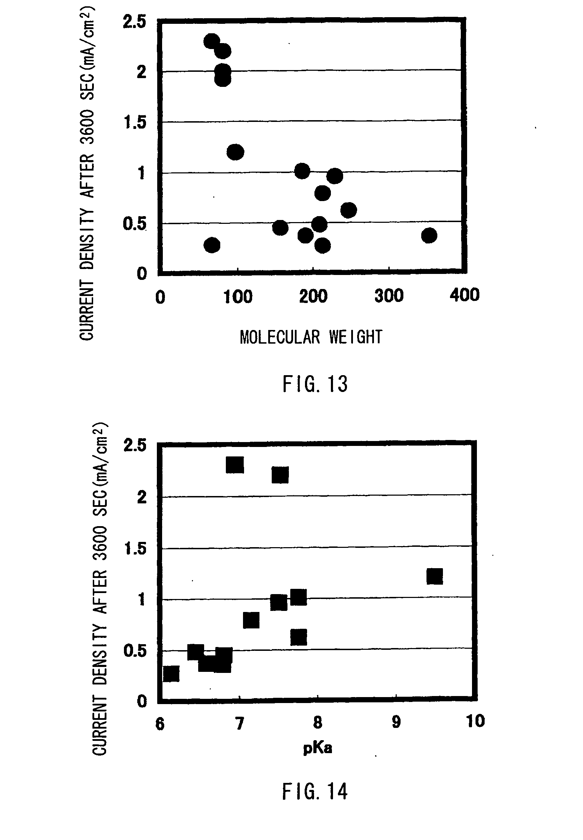

FIG. 13 and FIG. 14 illustrate a plot of current density after 3600 seconds in the case of using the buffer solutions illustrated in FIG. 11 and FIG. 12 with respect to molecular weight of the buffer substances and pKₐ.

Next, a description will be given of an example of a result of an experiment in which comparison of BOD activity was made in the case of using various buffer solutions. As the buffer solutions, the following buffer solutions were used.
2.0 M of imidazole/hydrochloric acid aqueous solution (solution obtained by neutralizing 2.0 M of imidazole with the use of hydrochloric acid to pH 7.0) (2.0 M of imidazole/hydrochloric acid buffer solution)
2.0 M of imidazole/acetic acid aqueous solution (solution obtained by neutralizing 2.0 M of imidazole with the use of acetic acid to pH 7.0) (2.0 M of imidazole/acetic acid buffer solution)
2.0 M of imidazole/phosphoric acid aqueous solution (solution obtained by neutralizing 2.0 M of imidazole with the use of phosphoric acid to pH 7.0) (2.0 M of imidazole/phosphoric acid buffer solution)
2.0 M of imidazole/sulfuric acid aqueous solution (solution obtained by neutralizing 2.0 M of imidazole with the use of sulfuric acid to pH 7.0) (2.0 M of imidazole/sulfuric acid buffer solution)
Measurement of BOD activity was made by using ABTS (2,2'-Azino-bis(3-ethylbenzothiazoline-6-sulfonic acid)diammonium salt) as a substrate, and tracing absorbance change of light with wavelength of 730 nm associated with reaction progress (resulting from increase of ABTS reactant). The measurement conditions are as illustrated in Table 2. The BOD concentration was adjusted so that absorbance change of light with wavelength of 730 nm became about from 0.01 to 0.2 per 1 minute in measuring activity. Reaction was started by adding an enzyme solution (from 5 to 20 µL both inclusive) to various buffer solutions (from 2980 to 2995 µL both inclusive) in Table 2 containing ABTS.

**[Table 3]**

| | |
|---|---|
| Buffer solution | 2.0 M of imidazole/hydrochloric acid aqueous solution (pH 7.0) |
| | 2.0 M of imidazole/acetic acid aqueous solution (pH 7.0) |
| | 2.0 M of imidazole/phosphoric acid aqueous solution (pH 7.0) |
| | 2.0 M of imidazole/sulfuric acid aqueous solution (pH 7.0) |
| ABTS concentration | 2 mM (final concentration) |
| O₂ concentration | Air saturation (210 µM: 25 deg C) |
| Reaction temperature | 25 deg C |

Table 4 illustrates the measurement result of enzyme activity as a relative activity value in the case where activity in the 2.0 M of imidazole/hydrochloric acid aqueous solution (pH 7.0) was 1.0.

**[Table 4]**

| Type of buffer solution | Relative activity value |
|---|---|
| 2.0 M of imidazole/hydrochloric acid aqueous solution (pH 7.0) | 1.0 |
| 2.0 M of imidazole/acetic acid aqueous solution (pH 7.0) | 2.1 |
| 2.0 M of imidazole/phosphoric acid aqueous solution (pH 7.0) | 3.7 |
| 2.0 M of imidazole/sulfuric acid aqueous solution (pH 7.0) | 11.2 |

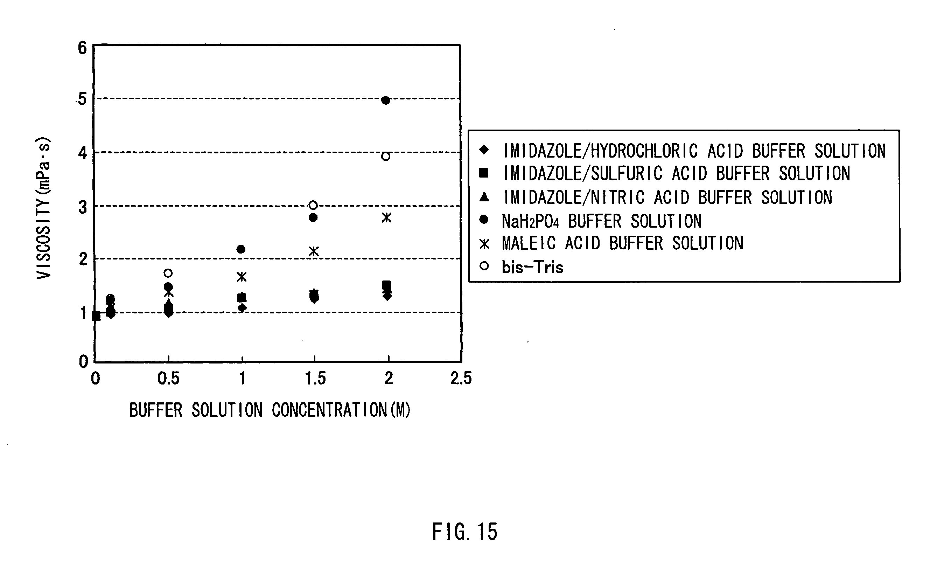

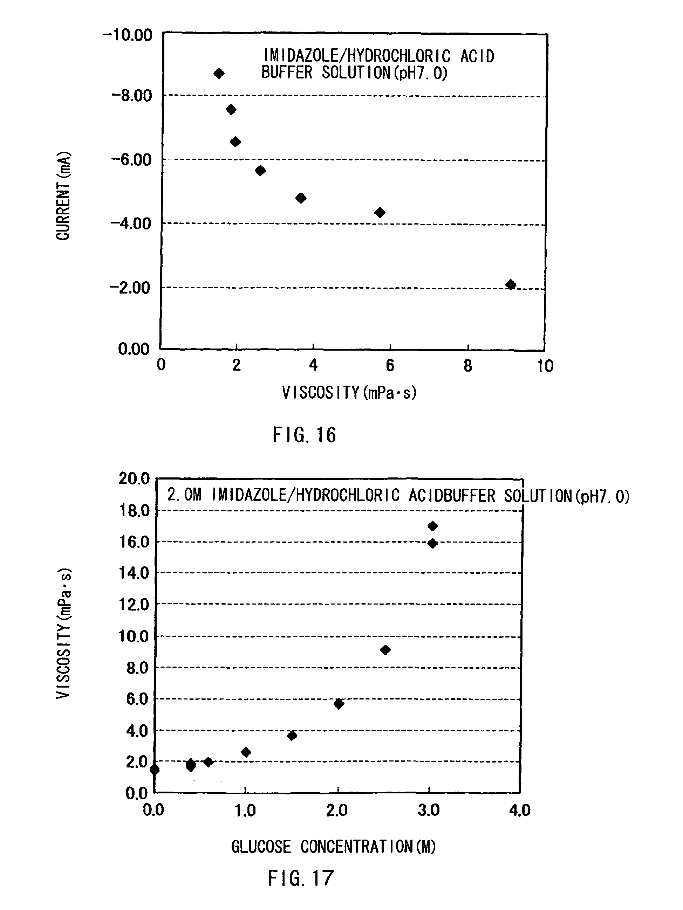

From Table 4, it was found that the enzyme activity in the case of using the imidazole/acetic acid aqueous solution, the imidazole/phosphoric acid aqueous solution, and the imidazole/sulfuric acid aqueous solution was higher than the enzyme activity in the case of using the imidazole/hydrochloric acid aqueous solution. In particular, the enzyme activity in the case of using the imidazole/ sulfuric acid aqueous solution was significantly high.
Next, a description will be given of a result of examining change of viscosity of buffer solutions according to the buffer solution concentrations. Measurement of viscosity was performed by using a viscometer at 24 deg C. FIG. 15 illustrates the results thereof. As a buffer solution, imidazole/hydrochloric acid buffer solution, imidazole/sulfuric acid buffer solution, imidazole/nitric acid buffer solution, NaH₂PO₄ buffer solution, maleic acid buffer solution, and bis-Tris buffer solution were used. As illustrated in FIG. 15, the viscosity of the NaH₂PO₄ buffer solution, the maleic acid buffer solution, and the bis-Tris buffer solution started to be increased intensely when the buffer solution concentration exceeded about 1.0 M, and was higher than 2.0 mPa·s when the buffer solution concentration exceeded about 1.5 M. Specially, the viscosity of the NaH₂PO₄ buffer solution and the bis-Tris buffer solution was significantly high, about 4.0 mPa·s or more when the buffer solution concentration became about 2.0 M. Meanwhile, the viscosity of the imidazole/hydrochloric acid buffer solution, the imidazole/sulfuric acid buffer solution, and the imidazole/nitric acid buffer solution was from 0.9 mPa·s to 2.0 mPa·s both inclusive in the case where the buffer solution concentration was from 0 M to 2.0 M both inclusive, and the viscosity thereof was sufficiently low, 1.3 mPa·s in the case where the buffer solution concentration was 2.0 M. In other words, even if the buffer solution concentration was increased, viscosity increase of the imidazole/hydrochloric acid buffer solution, the imidazole/sulfuric acid buffer solution, and the imidazole/nitric acid buffer solution was able to be inhibited.
Next, chronoamperometry was performed in a manner similar to the above-mentioned method by immobilizing BOD to the cathode 2 and using imidazole/hydrochloric acid buffer solution (pH 7.0), and change of a current after 3600 seconds according to the viscosity of the imidazole/hydrochloric acid buffer solution was examined. The results thereof will be hereinafter given. FIG. 16 illustrates the result. As illustrated in FIG. 16, it was found that in the case where the viscosity of the imidazole/hydrochloric acid buffer solution exceeded 2.0 mPa·s, the current was intensely decreased.
Next, a description will be given of a result of examining change of viscosity in the case where 2.0 M of imidazole/hydrochloric acid buffer solution (pH 7.0) was mixed with a glucose solution, and the glucose concentration was changed. FIG. 17 illustrates the result. As illustrated in FIG. 17, in the case where the glucose concentration exceeded 2.0 M, the viscosity started to be increased intensely.
Next, a description will be given of a reason why the viscosity of the buffer solution containing imidazole was lower than the viscosity of the NaH₂PO₄ buffer solution.

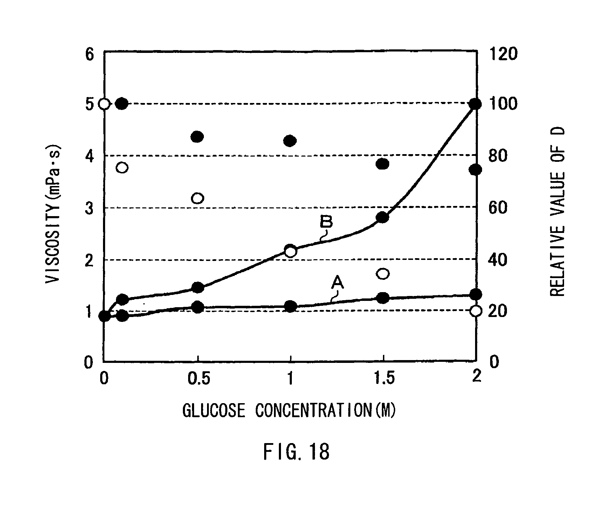

FIG. 18 illustrates change of the viscosity of the buffer solution containing imidazole and the viscosity of the NaH₂PO₄ buffer solution with respect to the buffer solution concentration. The viscosity of the buffer solution containing imidazole (curved line A) indicates an average value of the viscosity of the imidazole/hydrochloric acid buffer solution, the viscosity of the imidazole/sulfuric acid buffer solution, and the viscosity of the imidazole/nitric acid buffer solution illustrated in FIG. 15. The viscosity of the NaH₂PO₄ buffer solution (curved line B) is the same as that illustrated in FIG. 15. FIG. 18 also illustrates diffusion coefficient D of imidazole molecules in the buffer solution containing imidazole and diffusion coefficient D of NaH₂PO₄ molecules in the NaH₂PO₄ buffer solution (relative value with respect to the diffusion coefficient of NaH₂PO₄ molecules in the case where the concentration of the NaH₂PO₄ buffer solution was 2.0 M). The diffusion coefficient D was obtained by Stokes-Einstein formula, D=kBT/6πηr, where kB represents Boltzmann constant, T represents absolute temperature, η represents viscosity, and r represents a radius of a molecule.
As evidenced by FIG. 18, the viscosity of the buffer solution containing imidazole was lower than the viscosity of the NaH₂PO₄ buffer solution at least in the range up to the buffer solution concentration of 2.0 M. Such viscosity difference was reflected by contact angles. For example, a contact angle with respect to a carbon fiber electrode as a kind of carbon electrode was significantly small, 83.8 deg in the buffer solution containing imidazole (1 M), while a contact angle with respect to a carbon fiber electrode was large, 101.1 deg in the NaH₂PO₄ buffer solution (1 M). Since the contact angle of pure water with respect to the carbon fiber electrode was 93.9 deg, it was found that the contact angle of the buffer solution containing imidazole was significantly smaller than the contact angle of pure water. The fact that the contact angle of the buffer solution containing imidazole was significantly small meant that the buffer solution could be easily supplied to the electrodes, that is, to the cathode 2 and the anode 1.
As illustrated in FIG. 18, the diffusion coefficient of the NaH₂PO₄ molecules in the NaH₂PO₄ buffer solution (plot of the group of white circle not connected by a curved line in FIG. 18) was intensely decreased being reflected by the fact that the viscosity was intensely increased in association with increase of the buffer solution concentration. Meanwhile, the diffusion coefficient of the imidazole molecules in the buffer solution containing imidazole (plot of the group of black circle not connected by a curved line in FIG. 18) was slightly decreased being reflected by the fact that the viscosity increase in association with increase of the buffer solution concentration was significantly small. Conversely, it is able to be concluded that the reason why the viscosity of the buffer solution containing imidazole was kept low until high buffer solution concentration was the fact that the diffusion coefficient of the imidazole molecules in the buffer solution containing imidazole was kept large even if the buffer solution concentration was increased.

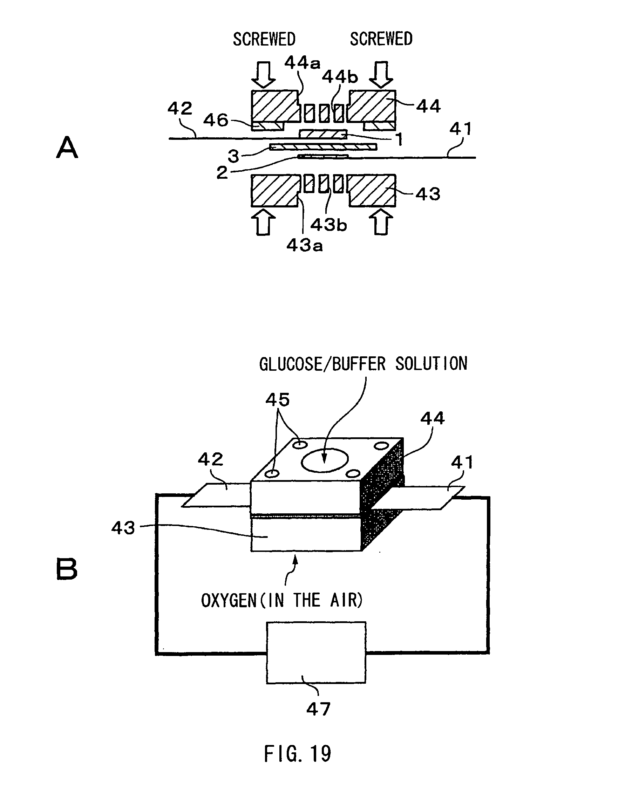

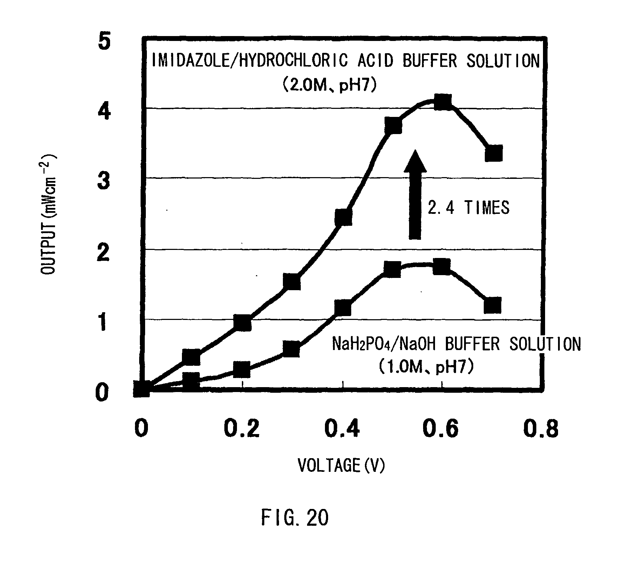

A specific structural example of the bio-fuel cell is illustrated in FIG. 19A and FIG. 19B.
As illustrated in FIG. 19A and FIG. 19B, the bio-fuel cell has a structure in which the anode 1 composed of an enzyme/electron mediator-immobilized carbon electrode and the cathode 2 composed of an enzyme/electron mediator-immobilized carbon electrode are opposed to each other with the electrolyte layer 3 containing a buffer substance in between. In the anode 1 composed of the enzyme/electron mediator-immobilized carbon electrode, the above-mentioned enzyme and the above-mentioned electron mediator are immobilized to a carbon felt having an area of 1 cm² by an immobilizer. In the cathode 2 composed of the enzyme/electron mediator-immobilized carbon electrode, the above-mentioned enzyme and the above-mentioned electron mediator are immobilized to a carbon felt having an area of 1 cm² by an immobilizer. The electrolyte layer 3 containing a buffer substance contains a compound containing an imidazole ring or 2-aminoethanol hydrochloride as a buffer substance. In this case, Ti current collectors 41 and 42 are respectively laid under the cathode 2 and on the anode 1 to facilitate current collection. Referential symbols 43 and 44 represent stationary plates. The stationary plates 43 and 44 are fastened to each other by a screw 45, and the cathode 2, the anode 1, the electrolyte layer 3, and the Ti current collectors 41 and 42 are sandwiched as a whole between the stationary plates 43 and 44. On one face (outer face) of the stationary plate 43, a circular concave section 43a for taking in the air is provided. On the bottom face of the concave section 43a, many holes 43b that penetrate through to the other face are provided. The holes 43b become an air supply route to the cathode 2. Meanwhile, on one face (outer face) of the stationary plate 44, a circular concave section 44a for feeding a fuel is provided. On the bottom face of the concave section 44a, many holes 44b that penetrate through to the other face are provided. The holes 44b become a fuel supply route to the anode 1. In the peripheral part of the other face of the stationary plate 44, a spacer 46 is provided. In the case where the stationary plates 43 and 44 are fastened on to each other by the screw 45, the distance between them becomes a given distance.
As illustrated in FIG. 19B, a load 47 was connected between the Ti current collectors 41 and 42, a glucose/buffer solution as a fuel was fed into the concave section 44a of the stationary plate 44, and power was generated. As a buffer solution, two types of buffer solutions that were 2.0 M of imidazole/hydrochloric acid buffer solution (pH 7) and 1.0 M of NaH₂PO₄/NaOH buffer solution (pH 7) were used. The glucose concentration was 0.4 M. The operation temperature was 25 deg C. FIG. 20 illustrates output characteristics. As illustrated in FIG. 20, the output (power density) in the case of using 2.0 M of imidazole/hydrochloric acid buffer solution as a buffer solution was about 2.4 times larger than the output (power density) in the case of using NaH₂PO₄/NaOH buffer solution.

As described above, according to the first embodiment, since the electrolyte layer 3 contains the compound containing an imidazole ring as a buffer substance, sufficient buffer ability is able to be obtained. Thus, even if protons are increased or decreased in the proton electrode or in the enzyme immobilized film by enzyme reaction through protons at the time of high output operation of the bio-fuel cell, sufficient buffer ability is able to be obtained. In result, shift of pH of the electrolyte around the enzyme from the optimal pH is able to be kept small sufficiently. Further, by adding one or more acids selected from the group consisting of acetic acid, phosphoric acid, and sulfuric acid in addition to the compound containing an imidazole ring, higher enzyme activity is able to be retained. Thereby, ability inherent in the enzyme is able to be sufficiently demonstrated, and electrode reaction by the enzyme, the coenzyme, and the electron mediator or the like is able to be efficiently and constantly initiated. Thereby, a highly efficient bio-fuel cell available for high output operation is able to be realized. The bio-fuel cell is suitably applied to a power source of various electronic devices, a movable body, a power generation system and the like.

### <2. Second embodiment>

### [Bio-fuel cell]

Next, a description will be given of a bio-fuel cell according to a second embodiment of the present invention.
In the bio-fuel cell, the electrolyte layer 3 has the same sign electric charge as that of an oxidant or a reductant of an electron mediator used for the cathode 2 and the anode 1. For example, at least surface on the cathode 2 side of the electrolyte layer 3 is negatively charged and has negative electric charge. Specifically, for example, polyanion having negative electric charge is contained in all or part of at least the section on the cathode 2 side of the electrolyte layer 3. It is suitable that as the polyanion, Nafion (product name, DuPont USA make) as an ion exchange resin having a fluorine-containing carbon sulfonate group is used.

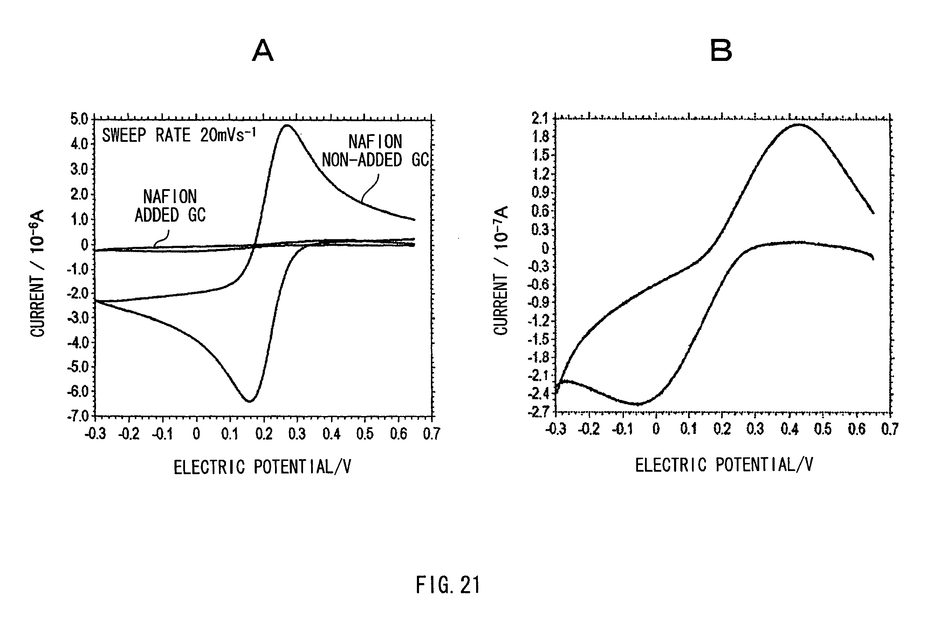

A description will be given of a result of a comparative experiment performed to verify a fact that an oxidant or a reductant of the electron mediator is able to be prevented from being transmitted through the electrolyte layer 3 in the case where the electrolyte layer 3 has the same sign electric charge as that of the oxidant or the reductant of the electron mediator.
First, commercially available two glassy carbon (GC) electrodes (diameter: 3 mm) were prepared, which were polished and washed. Next, one of the glassy carbon electrodes was added with 5 µl of commercially available Nafion emulsion (20%) as a polyanion, and the resultant was dried. Next, such two glassy carbon electrodes were soaked into 1 mM of hexacyanoferrate ion (multivalent ion) aqueous solution (50 mM of NaH₂PO₄/NaOH buffer solution, pH7), and cyclic voltammetry (CV) was performed at a sweep rate of 20 mVs⁻¹. The result is illustrated in FIG. 21A. FIG. 21B illustrates enlarged CV curved lines in the case where the glassy carbon electrode added with Nafion was used in FIG. 21A. As evidenced by FIG. 21A and FIG. 21B, in the glassy carbon electrode added with Nafion, the redox peak current originated in hexacyanoferrate ion as an electron mediator was one twentieth or less that of the glassy carbon electrode not added with Nafion. Such a result showed that the hexacyanoferrate ion as a multivalent anion that has negative electric charge as Nafion does was not diffused and transmitted through Nafion as a polyanion having negative electric charge.

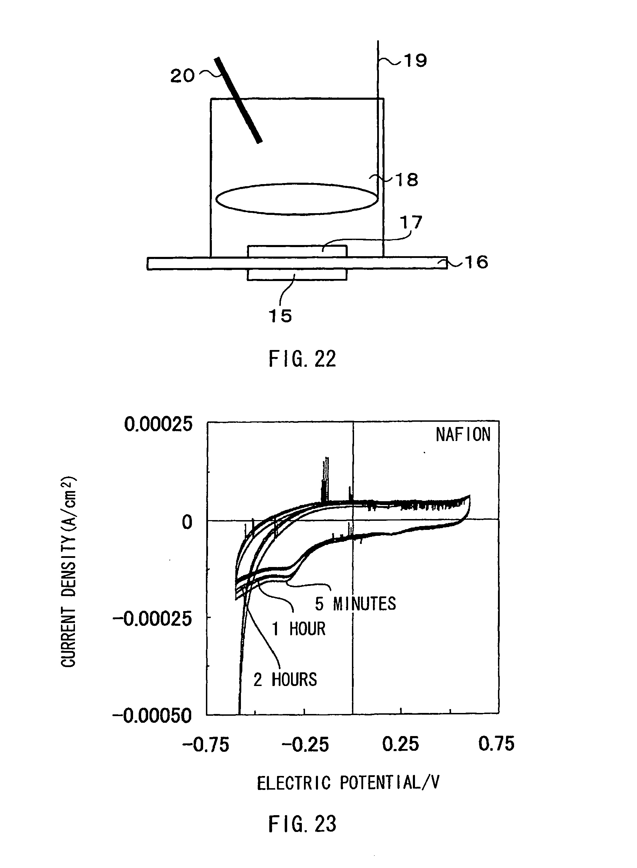

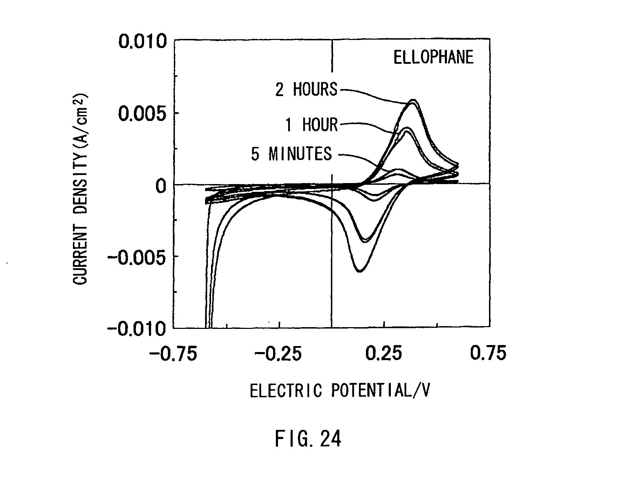

Next, as porous carbon, commercially available carbon felt (B0050, TORAY make) was used, and the carbon felt was cut into a 1 cm-square piece. The carbon felt was transfused with 80 µl of hexacyanoferrate ion (1 M), and the resultant was dried. Two pieces of the electrodes formed as above were layered, and the resultant was used as a test electrode. As illustrated in FIG. 22, a film-like separator 16 (corresponding to the electrolyte layer 3) was laid on a test electrode 15, and a working electrode 17 was provided to be opposed to the test electrode 15 with the separator 16 in between. As the working electrode 17, 1 cm-square piece cut out of commercially available carbon felt (B0050, TORAY make) was used. A substance obtained by dissolving hexacyanoferrate ion as an electron mediator in a buffer solution 18 composed of 0.4 M of NaH₂PO₄/NaOH (pH7) (a container for the buffer solution 18 was not illustrated) was contacted with the separator 16 and the working electrode 17. As the separator 16, cellophane not having electric charge and Nafion (pH7) as a polyanion having negative electric charge were used. 5 minutes, 1 hour, and 2 hours after contacting the separator 16 with the buffer solution 18 (electrolytic solution) in which hexacyanoferrate ion was dissolved, cyclic voltammetry was performed. Each redox peak value of the electron mediator transmitted from the test electrode 15 through the separator 16, that is, each redox peak value of hexacyanoferrate ion was compared to each other. A counter electrode 19 and a reference electrode 20 were soaked into the buffer solution 18. An electrochemical measurement equipment (not illustrated) was connected to the working electrode 17, the counter electrode 19, and the reference electrode 20. As the counter electrode 19, Pt line was used. As the reference electrode 20, Ag|AgCl was used. Measurement was performed under atmosphere pressure, and the measurement temperature was 25 deg C. The measurement result in the case of using Nafion as the separator 16 is illustrated in FIG. 23. Further, the measurement result in the case of using the cellophane as the separator 16 is illustrated in FIG. 24. As evidenced by FIG. 24, in the case where the cellophane was used as the separator 16, the redox peak of hexacyanoferrate ion was observed as early as 5 minutes after starting the measurement, and the redox peak value was increased as times goes by. Meanwhile, as illustrated in FIG. 23, in the case where Nafion was used as the separator 16, the redox peak of hexacyanoferrate ion was not observed even 2 hours after starting the measurement. Accordingly, it was confirmed that hexacyanoferrate ion was transmitted through the separator 16 in the case where the cellophane was used as the separator 16, while hexacyanoferrate ion was not transmitted through the separator 16 in the case where Nafion was used as the separator 16.

According to the second embodiment, in addition to an advantage similar to that of the first embodiment, the following advantage is able to be obtained. That is, the electrolyte layer 3 has the same sign electric charge as that of an oxidant or a reductant of the electron mediator used for the cathode 2 and the anode 1. Thus, it is able to effectively inhibit the electron mediator for one of the cathode 2 and the anode 1 from being transmitted through the electrolyte layer 3 and being moved to the other one of the cathode 2 and the anode 1. Thereby, lowering of output of the bio-fuel cell and lowering of electric capacity are able to be sufficiently inhibited.

### <3. Third embodiment>

### [Bio-fuel cell]

Next, a description will be given of a bio-fuel cell according to a third embodiment of the present invention.

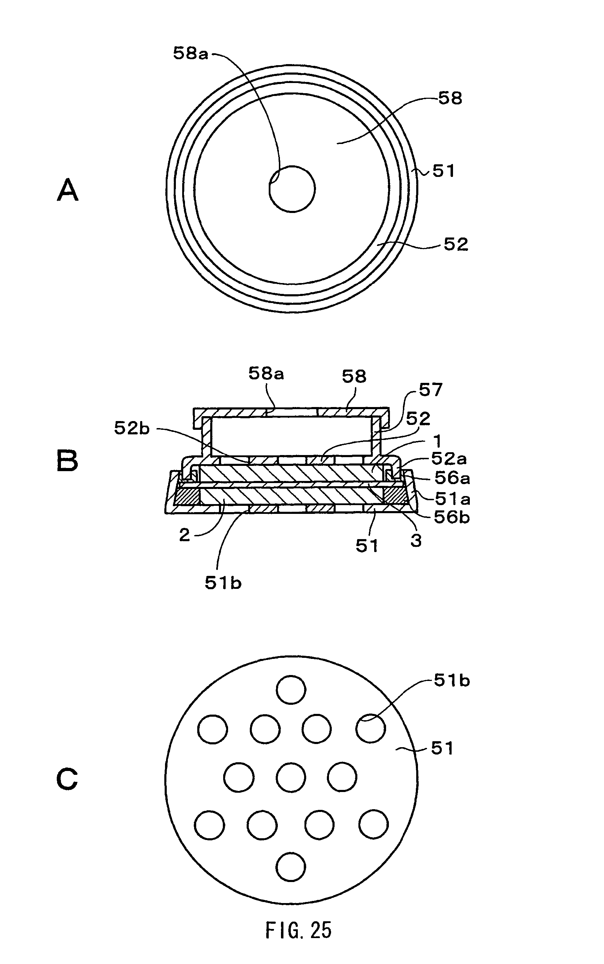

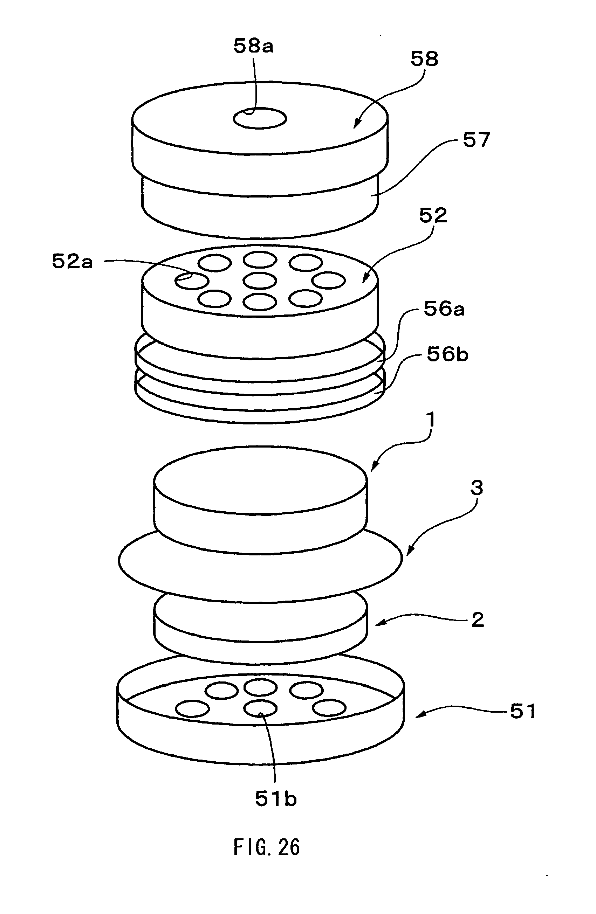

FIGS. 25A, 25B, 25C, and 26 illustrate the bio-fuel cell. FIGS. 25A, 25B, and 25C illustrate a top view, a cross sectional view, and a rear face view of the bio-fuel cell. FIG. 26 is an exploded perspective view illustrating disassembled respective components of the bio-fuel cell.

As illustrated in FIGS. 25A, 25B, 25C, and 26, in the bio-fuel cell, the cathode 2, the electrolyte layer 3, and the anode 1 are sandwiched between a cathode current collector 51 and an anode current collector 52 from above and beneath, and are contained in a space formed between the cathode current collector 51 and the anode current collector 52. Out of the cathode current collector 51, the anode current collector 52, the cathode 2, the electrolyte layer 3, and the anode 1, components adjacent to each other are contacted with each other. In this case, the cathode current collector 51, the anode current collector 52, the cathode 2, the electrolyte layer 3, and the anode 1 have a circular planar shape. The entire bio-fuel cell also has a circular planar shape.

The cathode current collector 51 is intended to collect a current generated in the cathode 2. The current is extracted outside from the cathode current collector 51. The anode current collector 52 is intended to collect a current generated in the anode 1. Though in general, the cathode current collector 51 and the anode current collector 52 are made of a metal, an alloy or the like, the material thereof is not limited thereto. The cathode current collector 51 has a flat and approximately cylindrical shape. The anode current collector 52 has a flat and approximately cylindrical shape as well. The edge of an outer circumferential section 51a of the cathode current collector 51 and an outer circumferential section 52a of the anode current collector 52 are caulked with a ring-like gasket 56a and a ring-like hydrophobic resin 56b, and therefore the space in which the cathode 2, the electrolyte layer 3, and the anode 1 are contained is formed. The gasket 56a is made of, for example, an insulative material such as silicon rubber. The hydrophobic resin 56b is made of, for example, polytetrafluoroethylene (PTFE) or the like. The hydrophobic resin 56b is provided to be contacted with the cathode 2, the cathode current collector 51, and the gasket 56a in the space surrounded by the cathode 2, the cathode current collector 51, and the gasket 56a. Excessive infiltration of the fuel into the cathode 2 side is able to be effectively inhibited by the hydrophobic resin 56b. The end section of the electrolyte layer 3 extends outside of the cathode 2 and the anode 1, and is sandwiched between the gasket 56a and the hydrophobic resin 56b. The cathode current collector 51 has a plurality of oxidant supply ports 51b on the whole area of the bottom face thereof. The cathode 2 is exposed inside of the oxidant supply ports 51b. Though FIG. 21C and FIG. 22 illustrate 13 pieces of circular oxidant supply ports 51b, such illustration is an only example. The number, the shape, the size, and the arrangement of the oxidant supply ports 51b are able to be selected as appropriate. The anode current collector 52 has a plurality of fuel supply ports 52b on the whole area of the top face thereof as well. The anode 1 is exposed inside of the fuel supply ports 52b. Though FIG. 22 illustrates seven circular fuel supply ports 52b, such illustration is an only example. The number, the shape, the size, and the arrangement of the fuel supply ports 52b are able to be selected as appropriate.

The anode current collector 52 has a cylindrical fuel tank 57 on the side opposite to the anode 1 with respect to the anode current collector 52. The fuel tank 57 is formed integrally with the anode current collector 52. A fuel to be used (not illustrated) such as glucose solution and glucose solution further added with an electrolyte is contained in the fuel tank 57. The fuel tank 57 is attached with a removable cylindrical cover 58. The cover 58 is fitted in the fuel tank 57 or is fastened thereto by a screw. In the central section of the cover 58, a circular fuel supply port 58a is formed. The fuel supply port 58a is hermetically sealed by, for example, sticking a seal (not illustrated).
Structures other than the foregoing description of the bio-fuel cell are similar to those of the first embodiment as long as the structures do not work against the characteristics.

### [Method of manufacturing bio-fuel cell]

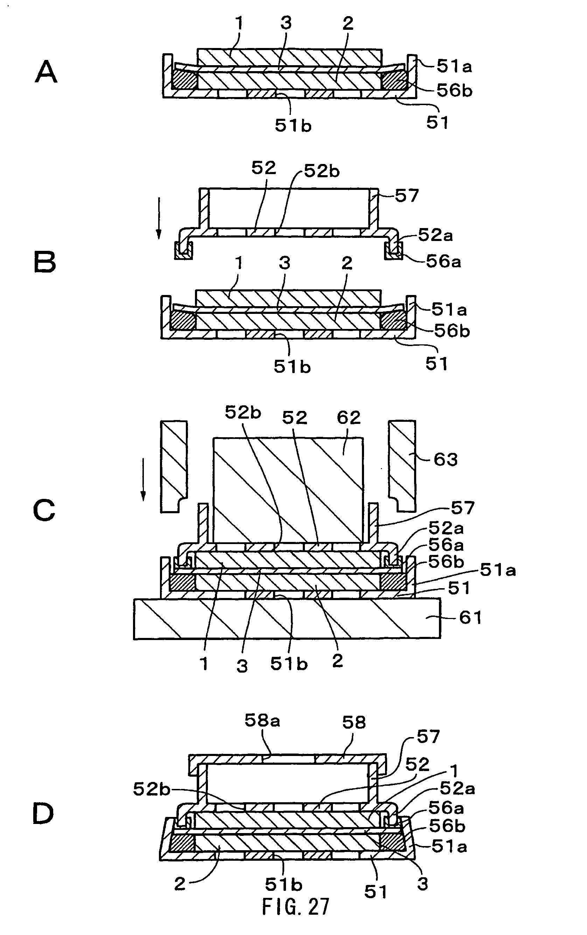

Next, a description will be given of an example of a method of manufacturing the bio-fuel cell. The manufacturing method is illustrated in FIGS. 27A to 27D.
As illustrated in FIG. 27A, first, the cylindrical cathode current collector 51 in which one end is opened is prepared. The plurality of oxidant supply ports 51b are formed on the whole area of the bottom face of the cathode current collector 51. The ring-like hydrophobic resin 56b is laid on the outer circumferential section of the inner bottom face of the cathode current collector 51. On the central section of the bottom face, the cathode 2, the electrolyte layer 3, and the anode 1 are sequentially layered.

Meanwhile, as illustrated in FIG. 27B, the integrated body in which the cylindrical fuel tank 57 is formed on the cylindrical anode current collector 52 with one end opened is prepared. The plurality of fuel supply ports 52b are formed on the whole area of the anode current collector 52. The gasket 56a having a U-shaped cross sectional shape is attached to the edge of the outer circumferential face of the anode current collector 52. The anode current collector 52 with the open section side downward is laid on the anode 1. The cathode 2, the electrolyte layer 3, and the anode 1 are sandwiched between the cathode current collector 51 and the anode current collector 52.

Next, as illustrated in FIG. 27C, the body in which the cathode 2, the electrolyte layer 3, and the anode 1 are sandwiched between the cathode current collector 51 and the anode current collector 52 is laid on a table 61 of a caulking machine. The anode current collector 52 is pressed by a presser member 62, and therefore components adjacent to each other out of the cathode current collector 51, the cathode 2, the electrolyte layer 3, the anode 1, and the anode current collector 52 are contacted with each other. In this state, a calking tool 63 is lowered, and the edge of the outer circumferential section 51b of the cathode current collector 51 and the outer circumferential section 52b of the anode current collector 52 are caulked with the gasket 56a and the hydrophobic resin 56b. Such caulking is made so that the gasket 56a is gradually crushed to prevent a gap between the cathode current collector 51 and the gasket 56a and a gap between the anode current collector 52 and the gasket 56a. At this time, the hydrophobic resin 56b is also gradually compressed, and therefore the cathode 2, the cathode current collector 51, and the gasket 56a are contacted with each other. Thereby, the space to contain the cathode 2, the electrolyte layer 3, and the anode 1 is formed inside the cathode current collector 51 and the anode current collector 52 in a state that the cathode current collector 51 and the anode current collector 52 are electrically insulated from each other by the gasket 56a. After that, the calking tool 63 is lifted.

Accordingly, as illustrated in FIG. 27D, the bio-fuel cell in which the cathode 2, the electrolyte layer 3, and the anode 1 are contained in the space formed between the cathode current collector 51 and the anode current collector 52 is manufactured.
Next, the cover 58 is attached to the fuel tank 57, and the fuel and the electrolyte are injected through the fuel supply port 58a of the cover 58. After that, the fuel supply port 58a is closed by, for example, sticking a seal. However, the fuel and the electrolyte may be injected into the fuel tank 57 in the step illustrated in FIG. 27B.

In the bio-fuel cell, in the case where, for example, a glucose solution is used as a fuel injected into the fuel tank 57, in the anode 1, the supplied glucose is degraded by the enzyme, electrons are extracted and H⁺ is generated. In the cathode 2, water is generated from H⁺ transported from the anode 1 through the electrolyte layer 3, electrons sent from the anode 1 through the external circuit, and, for example, oxygen in the air. Accordingly, output voltage is obtained between the cathode current collector 51 and the anode current collector 52.

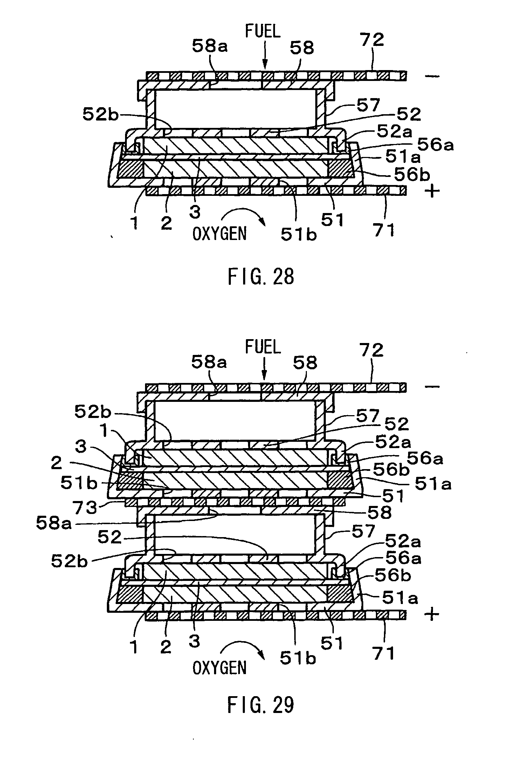

As illustrated in FIG. 28, mesh electrodes 71 and 72 may be respectively formed on the cathode current collector 51 and the anode current collector 52 of the bio-fuel cell. In this case, external air enters into the oxidant supply ports 51 b of the current collector 51 through holes of the mesh electrode 71, and the fuel enters into the fuel tank 57 through the fuel supply ports 58a of the cover 58 through holes of the mesh electrode 72.

FIG. 29 illustrates a case that two bio-fuel cells are connected in series. In this case, a mesh electrode 73 is sandwiched between the cathode current collector 51 of one bio-fuel cell (the upper bio-fuel cell in the figure) and the cover 58 of the other bio-fuel cell (the lower bio-fuel cell in the figure). In this case, external air enters into the oxidant supply ports 51b of the cathode current collector 51 through holes of the mesh electrode 73. The fuel is able to be supplied by a fuel supply system as well.

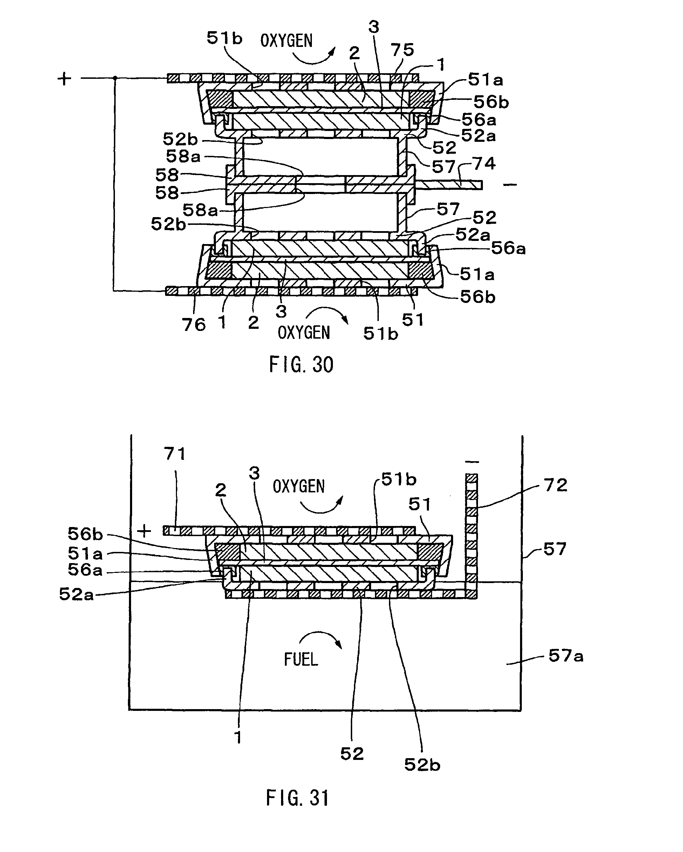

FIG. 30 illustrates a case that two bio-fuel cells are connected in parallel. In this case, the fuel tank 57 of one bio-fuel cell (the upper bio-fuel cell in the figure) and the fuel tank 57 of the other bio-fuel cell (the lower bio-fuel cell in the figure) are contacted with each other so that the respective fuel supply ports 58a of the respective covers 58 correspond with each other. An electrode 74 is led out from the side face of the fuel tanks 57. Further, mesh electrodes 75 and 76 are respectively formed on the cathode current collector 51 of the foregoing one bio-fuel cell and the cathode current collector 51 of the foregoing other bio-fuel cell. The mesh electrodes 75 and 76 are connected with each other. External air enters into the oxidant supply ports 51b of the cathode current collector 51 through the holes of the mesh electrodes 75 and 76.

According to the third embodiment, an advantage similar to that of the first embodiment is able to be obtained in a coin type bio-fuel cell and a button type bio-fuel cell except for the fuel tank 57. Further, in the bio-fuel cell, the cathode 2, the electrolyte layer 3, and the anode 1 are sandwiched between the cathode current collector 51 and the anode current collector 52, and the edge of the outer circumferential section 51a of the cathode current collector 51 and the outer circumferential section 52a of the anode current collector 52 are caulked with the gasket 56. Thereby, in the bio-fuel cell, the respective components are able to be uniformly contacted with each other. Thus, output variation is able to be prevented, and the fuel and the battery solution such as the electrolyte are able to be prevented from being leaked from an interface between the respective components. Further, the manufacturing steps of the bio-fuel cell are simple. Further, the bio-fuel cell is able to be easily downsized. Further, in the bio-fuel cell, in the case where the glucose solution or starch is used as a fuel, and pH of the electrolyte used is selected as a value in the vicinity of 7 (neutrality), safety is secured in case of external leakage of the fuel or the electrolyte.
Further, in the air cell practically used currently, it is necessary to add a fuel and an electrolyte at the time of manufacturing thereof, and it is difficult to add the fuel and the electrolyte after manufacturing the air cell. Meanwhile, in the bio-fuel cell, it is able to add the fuel and the electrolyte after manufacturing the bio-fuel cell. Thus, the bio-fuel cell is more easily manufactured than the air cell practically and currently used is.

### <4. Fourth embodiment>

### [Bio-fuel cell]

Next, a description will be given of a bio-fuel cell according to a fourth embodiment of the present invention.
As illustrated in FIG. 31, in the fourth embodiment, a bio-fuel cell obtained by excluding the fuel tank 57 provided integrally with the anode current collector 52 from the bio fuel cell according to the third embodiment in which the mesh electrodes 71 and 72 are respectively formed on the cathode current collector 51 and the anode current collector 52 is used. The bio-fuel cell is used in a state that the bio-fuel cell is floated on a fuel 57a contained in the open fuel tank 57 with the anode 1 side downward and the cathode 2 side upward.
Structures other than the foregoing description of the bio-fuel cell of the fourth embodiment are similar to those of the first to the third embodiments as long as the structures do not work against the characteristics.
According to the fourth embodiment, advantages similar to those of the first to the third embodiments are able to be obtained.

### <5. Fifth embodiment>

### [Bio-fuel cell]

Next, a description will be given of a bio-fuel cell according to a fifth embodiment of the present invention. While the bio-fuel cell according to the third embodiment is the coin type bio-fuel cell or the button type bio-fuel cell, the bio-fuel cell in this embodiment is a cylindrical type bio-fuel cell.

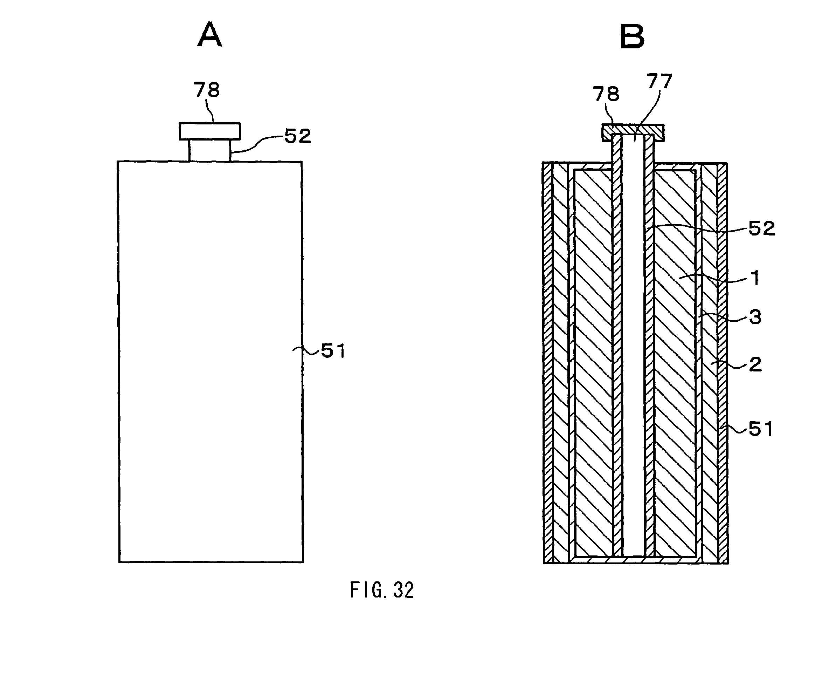

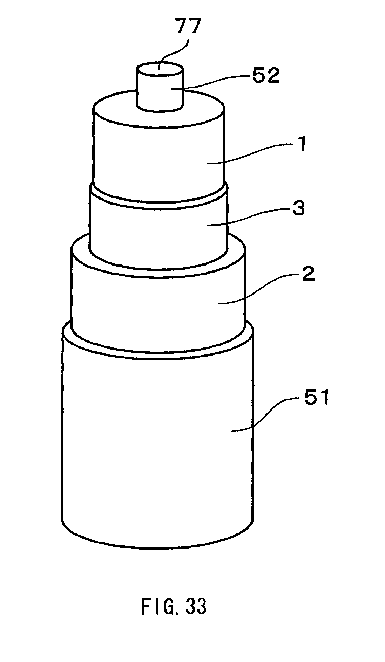

FIGS. 32A, 32B, and 33 illustrate the bio-fuel cell. FIG. 32A is an elevation view of the bio-fuel cell, FIG. 32B is a vertical cross sectional view of the bio-fuel cell, and FIG. 33 is an exploded perspective view illustrating respective disassembled components of the bio-fuel cell.

As illustrated in FIGS. 32A, 32B, and 33, in the bio-fuel cell, the cylindrical anode current collector 52, the cylindrical anode 1, the cylindrical electrolyte layer 3, the cylindrical cathode 2, and the cylindrical cathode current collector 51 are sequentially provided in the outer circumference of a cylindrical fuel retention section 77. In this case, the fuel retention section 77 is composed of a space surrounded by the cylindrical anode current collector 52. One end of the fuel retention section 77 is protruded outside, and such one end is attached with a cover 78. Though not illustrated, in the anode current collector 52 in the outer circumference of the fuel retention section 77, the plurality of fuel supply ports 52b are formed on the whole area of the face thereof. Further, the electrolyte layer 3 is in a state of a pouch that envelops the anode 1 and the anode current collector 52. A section between the electrolyte layer 3 and the anode current collector 52 in one end of the fuel retention section 77 is hermetically sealed by, for example, a seal member (not illustrated) to prevent the fuel from being leaked outside from such a section.

In the bio-fuel cell, a fuel and an electrolyte are injected into the fuel retention section 77. The fuel and the electrolyte pass through the fuel supply port 52b of the anode current collector 52, reach the anode 1, are permeated into a void section of the anode 1, and therefore are able to be stored in the anode 1. To increase the fuel amount capable of being stored in the anode 1, the void ratio of the anode 1 is desirably, for example, 60% or more, but the void ratio is not limited thereto.

In the bio-fuel cell, a vapor-liquid separation layer may be provided on the outer circumferential face of the cathode current collector 51 in order to improve decay durability. As a material of the vapor-liquid separation layer, for example, a waterproof moisture permeable raw material (a combined raw material of a film obtained by stretching polytetrafluoroethylene and polyurethane polymer) (for example, Gore-Tex (product name), W.L. Gore & Associates, Inc. make) is used. To uniformly contact the respective components of the bio-fuel cell with each other, it is suitable that an elastic rubber (band-like rubber or sheet-like rubber) having mesh structure through which external air is able to permeate is wound around outside or inside of the vapor-liquid separation layer to fasten the entire components of the bio-fuel cell.
Structures other than the foregoing description of the bio-fuel cell of the fifth embodiment are similar to those of the first to the third embodiments as long as the structures do not work against the characteristics.
According to the fifth embodiment, advantages similar to those of the first to the third embodiments are able to be obtained.

### <6. Sixth embodiment>

### [Bio-fuel cell]

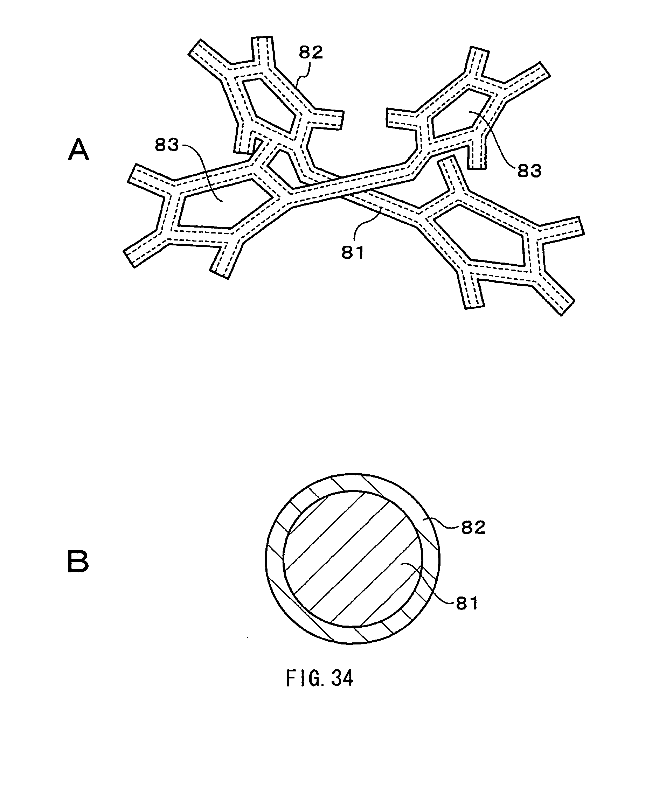

Next, a description will be given of a bio-fuel cell according to a sixth embodiment of the present invention.
The bio-fuel cell according to the sixth embodiment has a structure similar to that of the bio-fuel cell according to the first embodiment, except that a porous conductive material as illustrated in FIGS. 34A and 34B is used as a material of the electrode 11 of the anode 1.
FIG. 34A schematically illustrates a structure of the porous conductive material, and FIG. 34B is a cross sectional view of a skeleton section of the porous conductive material. As illustrated in FIGS. 34A and 34B, the porous conductive material is composed of a skeleton 81 made of a porous material having a three dimensional mesh structure and a carbon material 82 covering the surface of the skeleton 81. The porous conductive material has the three dimensional mesh structure in which many pores 83 surrounded by the carbon material 82 correspond to mesh. In this case, the respective pores 83 communicate with each other. The form of the carbon material 82 is not limited, and any shape such as fiber state (needle state) and granular state is able to be adopted.

As the skeleton 81 made of the porous material, a foamed metal or a foamed alloy such as foamed nickel is used. The porosity ratio of the skeleton 81 is generally 85% or more, and is more generally 90% or more. The pore diameter thereof is generally, for example, from 10 nm to 1 mm both inclusive, is more generally from 10 nm to 600 µm both inclusive, is much more generally from 1 to 600 µm both inclusive, is typically from 50 to 300 µm both inclusive, and is more typically from 100 to 250 µm both inclusive. As the carbon material 82, though a high conductive material such as Ketjen black is preferable, a functional carbon material such as carbon nanotube and fullerene may be used.
The porosity ratio of the porous conductive material is generally 80% or more, and is more generally 90% or more. The diameter of the pore 83 is generally, for example, from 9 nm to 1 mm both inclusive, is more generally from 9 nm to 600 µm both inclusive, is much more generally from 1 to 600 µm both inclusive, is typically from 30 to 400 µm both inclusive, and is more typically from 80 to 230 µm both inclusive.

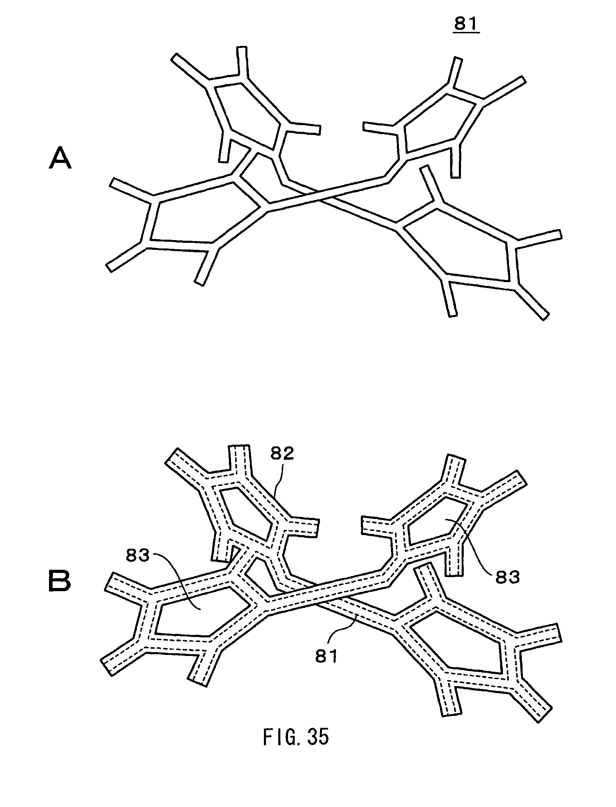

Next, a description will be given of a method of manufacturing the porous conductive material.
As illustrated in FIG. 35A, first, the skeleton 81 made of a foamed metal or a foamed alloy (for example, foamed nickel) is prepared.
Next, as illustrated in FIG. 35B, the surface of the skeleton 81 made of the foamed metal or the foamed alloy is coated with the carbon material 82. As coating method thereof, a known method is able to be used. As an example, there is a method that the surface of the skeleton 81 is coated with the carbon material 82 by spraying emulsion containing carbon powder, an appropriate binder and the like with the use of a spray onto the surface of the skeleton 81. The coating thickness of the carbon material 82 is determined according to the porosity ratio and the pore diameter needed for the porous conductive material with the porosity ratio and the pore diameter of the skeleton 81 made of the foamed metal or the foamed alloy in mind. Coating is made so that the many pores 83 surrounded by the carbon material 82 are communicated with each other.
Accordingly, the intended porous conductive material is manufactured.

According to the sixth embodiment, the porous conductive material in which the surface of the skeleton 81 made of the foamed metal or the foamed alloy is coated with the carbon material 82 has the pore 83 with a sufficiently large diameter and the rough three dimensional mesh structure. In addition, the porous conductive material has high intensity and high conductivity, and has a sufficient surface area. Thus, in the anode 1 formed from the electrode 81 formed by using the porous conductive material on which an enzyme, a coenzyme, an electron mediator and the like are immobilized, enzyme metabolic reaction thereon is able to be effectively initiated. In addition, the anode 1 is able to effectively capture enzyme reaction phenomenon generated in the vicinity of the electrode 11 as an electric signal, is stable without relation to usage environment, and is able to realize a high-performance bio-fuel cell.

### <7. Seventh embodiment>

### [Bio-fuel cell]

Next, a description will be given of a bio-fuel cell according to a seventh embodiment of the present invention.
In the bio-fuel cell, starch as a polysaccharide is used as a fuel. Further, in association with using starch as a fuel, glucoamylase as a degradation enzyme to degrade starch to glucose is immobilized to the anode 11.
In the bio-fuel cell, in the case where starch is supplied as a fuel to the anode 1 side, the starch is hydrolyzed to glucose by glucoamylase, and the glucose is degraded by glucose dehydrogenase. In association with oxidation reaction in the degradation process, NAD⁺ is reduced and NADH is generated. Such NADH is oxidized by diaphorase and is separated into two electrons, NAD⁺, and H⁺. Thus, two electrons and two H⁺ are generated by one stage oxidation reaction per one molecule of glucose. In two stage oxidation reaction, four electrons and four H⁺ are generated in total. The electrons generated as above are delivered to the electrode 11 of the anode 1, and H⁺ are moved to the cathode 2 through the electrolyte layer 3. In the cathode 2, such H⁺ is reacted with externally supplied oxygen and the electrons sent from the anode 1 through an external circuit, and therefore H₂O is generated.
Structures other than the foregoing description of the bio-fuel cell are similar to those of the bio-fuel cell according to the first embodiment.
According to the seventh embodiment, an advantage similar to that of the first embodiment is able to be obtained. In addition, since starch is used as a fuel, an advantage that the power generation amount is able to be increased more than in a case of using glucose as a fuel is able to be obtained.

The present invention has been described with reference to the embodiments. However, the present invention is not limited to the foregoing embodiments, and various modifications based on technical idea of the present invention may be made.
For example, the numerical values, the structures, the constructions, the shapes, the materials and the like described in the foregoing embodiments are only examples, and numerical values, structures, constructions, shapes, materials and the like different from those may be used according to needs.

In the existing bio-fuel cells, selecting the electron mediator that plays a role to deliver and receive electrons between the enzyme and the electrode largely has affected battery output. In other words, there has been a problem as follows. In the case where an electron mediator with small free energy difference with respect to the substrate is selected in order to obtain high output voltage of the battery, a sufficient current value is not able to be obtained. On the contrary, in the case where an electron mediator with high free energy difference with respect to the substrate is selected, a current capacity becomes small. Such a problem is able to be resolved by concurrently using two or more types of electron mediators each having different redox electric potential for the anode 1 and/or the cathode 2 so that both high output voltage and a high current are able to be obtained respectively. In this case, each redox electric potential of the two or more types of electron mediators is suitably different from each other by 50 mV or more, is more suitably different from each other by 100 mV or more, and is much more suitably different from each other by 200 mV or more at pH 7.0. By concurrently using two or more types of electron mediators immobilized to the anode 1 or the cathode 2 as above, battery work at high electric potential with small energy loss is able to be realized at the time of request of low output. Thus, a bio-fuel cell that is able to allow high output with high energy loss at the time of request of high output is able to be realized.

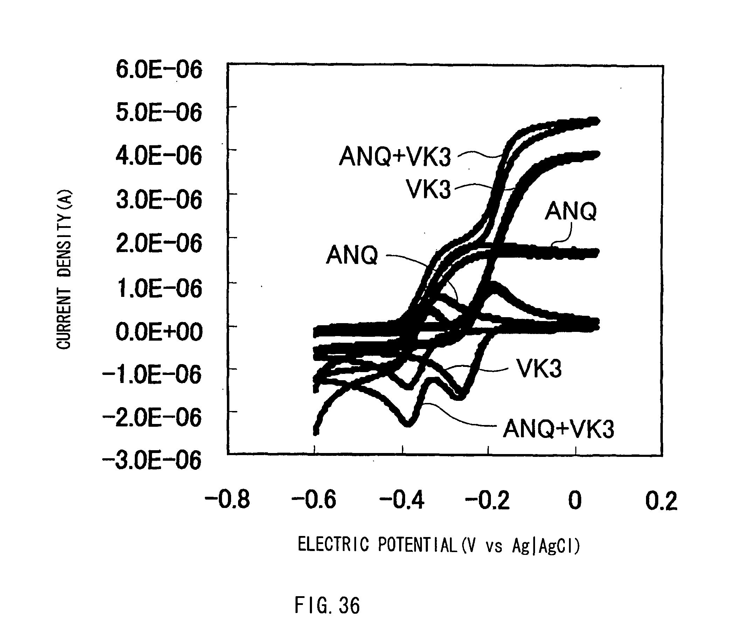

FIG. 36 illustrates results from an experiment in which 100 µM of only VK3 (vitamin K3), 100 µM of only ANQ, or each 100 µM of VK3 and ANQ were added to 0.1 M of NaH₂PO₄/NaOH buffer solution (pH7) and cyclic voltammetry was performed. The redox electric potential at pH7 of VK3 and ANQ was respectively -0.22 V and -0.33 V (v.s. Ag|AgCl), and each redox electric potential was different from each other by 0.11 V (110 mV). After that, adjustment was made so that concentration of NADH in the solution was 5 mM and concentration of enzyme diaphorase in the solution was 0.16 µM, and cyclic voltammetry was performed. The result thereof is also illustrated in FIG. 36. As evidenced by FIG. 36, in the case where VK3 and ANQ each having redox electric potential different by 110 mV at pH7 were used as an electron mediator, a higher output voltage and a higher output current value were able to be realized than in a case that VK3 and ANQ were used individually.

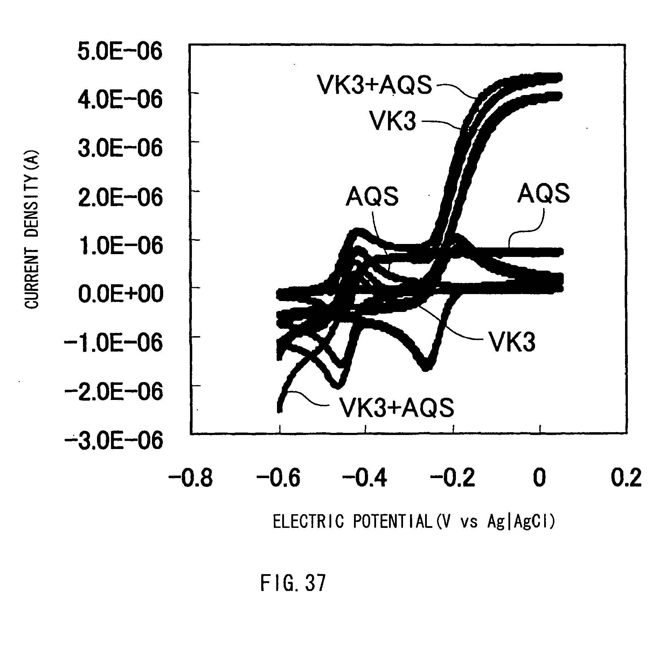

FIG. 37 illustrates results from an experiment in which 100 µM of only VK3, 100 µM of only AQS, or each 100 µM of VK3 and AQS were added to 0.1 M of NaH₂PO₄/NaOH buffer solution (pH7) and cyclic voltammetry was performed. The redox electric potential at pH7 of VK3 and AQS was respectively -0.22 V and -0.42 V (v.s. Ag|AgCl), and each redox electric potential was different from each other by 0.2 V (200 mV). After that, adjustment was made so that concentration of NADH in the solution was 5 mM and concentration of enzyme diaphorase in the solution was 0.16 µM, and cyclic voltammetry was performed. The result thereof is also illustrated in FIG. 37. As evidenced by FIG. 37, in the case where VK3 and AQS each having redox electric potential different by 200 mV at pH7 were used as an electron mediator, a higher output voltage and a higher output current value were able to be realized than in a case that VK3 and AQS were used individually.

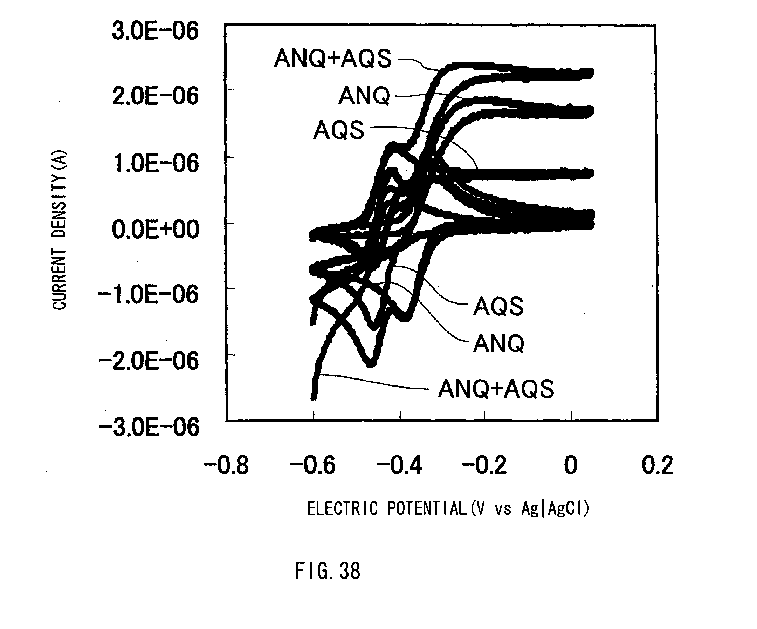

FIG. 38 illustrates results from an experiment in which 100 µM of only ANQ, 100 µM of only AQS, or each 100 µM of ANQ and AQS were added to 0.1 M of NaH₂PO₄NaOH buffer solution (pH7) and cyclic voltammetry was performed. The redox electric potential at pH7 of ANQ and AQS was respectively -0.33 V and -0.42 V (v.s. Ag|AgCl), and each redox electric potential was different from each other by 0.09 V (90 mV). After that, adjustment was made so that concentration of NADH in the solution was 5 mM and concentration of enzyme diaphorase in the solution was 0.16 µM, and cyclic voltammetry was performed. The result thereof is also illustrated in FIG. 38. As evidenced by FIG. 38, in the case where ANQ and AQS each having redox electric potential different by 90 mV at pH7 were used as an electron mediator, a higher output voltage and a higher output current value were able to be realized than in a case that ANQ and AQS were used individually.

Using the two or more types of electron mediators each having different redox electric potential as above is effectively applied not only to the bio-fuel cell using the enzyme but also to a case of using an electron mediator in a bio-fuel cell using a microorganism or a cell. More generally, using the two or more types of electron mediators each having different redox electric potential is effectively applied to electrode reaction usage equipment using the electron mediator (a bio-fuel cell, a bio sensor, a bio reactor and the like) generally.

## Claims

1. A fuel cell having a structure in which a cathode and an anode are opposed to each other with an electrolyte containing a buffer substance in between,
wherein an enzyme is immobilized to one or both of the cathode and the anode, and a compound containing an imidazole ring is contained in the buffer substance, and one or more acids selected from the group consisting of acetic acid, phosphoric acid, and sulfuric acid are added.

2. The fuel cell according to claim 1, wherein concentration of the buffer substance is from 0.2 M to 2.5 M both inclusive.

3. The fuel cell according to claim 2, wherein viscosity of a buffer solution containing the buffer substance is from 0.9 mPa·s to 2.0 mPa·s both inclusive.

4. The fuel cell according to claim 2, wherein the enzyme contains an oxygen reduction enzyme immobilized to the cathode.

5. The fuel cell according to claim 4, wherein the oxygen reduction enzyme is bilirubin oxidase.

6. The fuel cell according to claim 1, wherein in addition to the enzyme, an electron mediator is immobilized to one or both of the cathode and the anode.

7. The fuel cell according to claim 1, wherein the enzyme contains an oxidase that is immobilized to the anode and promotes oxidation of a monomeric sugar and degrades the same.

8. The fuel cell according to claim 7, wherein the enzyme contains a coenzyme oxidase that returns a coenzyme reduced in association with the oxidation of the monomeric sugars to an oxidant and that delivers an electron to the anode through an electron mediator.

9. The fuel cell according to claim 8, wherein the oxidant of the coenzyme is NAD⁺, and the coenzyme oxidase is diaphorase.

10. The fuel cell according to claim 7, wherein the oxidase is NAD⁺ dependent glucose dehydrogenase.

11. The fuel cell according to claim 1, wherein the enzyme contains a degradation enzyme that is immobilized to the anode and that promotes degradation of a polysaccharide to generate a monomeric sugar and an oxidase that promotes oxidation of the generated monomeric sugar and degrades the same.

12. The fuel cell according to claim 11, wherein the degradation enzyme is glucoamylase, and the oxidase is NAD⁺ dependent glucose dehydrogenase.

13. An electronic device having one or a plurality of fuel cells, wherein one or more of the fuel cells have a structure in which a cathode and an anode are opposed to each other with an electrolyte containing a buffer substance in between,
an enzyme is immobilized to one or both of the cathode and the anode, and
a compound containing an imidazole ring is contained in the buffer substance, and one or more acids selected from the group consisting of acetic acid, phosphoric acid, and sulfuric acid are added.

14. A buffer solution for a fuel cell used for a fuel cell, which has a structure in which a cathode and an anode are opposed to each other with an electrolyte containing a buffer substance in between, and in which an enzyme is immobilized to one or both of the cathode and the anode, wherein
a compound containing an imidazole ring is contained in the buffer substance, and one or more acids selected from the group consisting of acetic acid, phosphoric acid, and sulfuric acid are added.
